(19) Europäisches Patentamt
European Patent Office
Office européen des brevets

(11) EP 4 520 749 A1

(12) EUROPEAN PATENT APPLICATION
published in accordance with Art. 153(4) EPC

(43) Date of publication:
**12.03.2025 Bulletin 2025/11**

(21) Application number: **23806623.7**

(22) Date of filing: **31.03.2023**

(51) International Patent Classification (IPC):
***C07D 207/27*** (2006.01) ***A61Q 17/04*** (2006.01)
***A61Q 19/02*** (2006.01) ***A61K 8/00*** (2006.01)
***C07C 37/00*** (2006.01)

(52) Cooperative Patent Classification (CPC):
(C-Sets available)
**C07C 39/15; A61K 8/347; A61K 8/4906; A61K 8/4913; A61K 8/4926; A61K 31/085; A61K 31/4015; A61K 31/4412; A61K 31/55; A61P 17/00; A61Q 19/02; C07C 37/11; C07C 43/23; C07D 207/27; C07D 211/76;** (Cont.)

(86) International application number:
**PCT/CN2023/085442**

(87) International publication number:
**WO 2023/221665 (23.11.2023 Gazette 2023/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.05.2022 CN 202210534437**
**24.11.2022 CN 202211487425**

(71) Applicant: **Purpana (Beijing) Technologies Co., Ltd**
**Beijing, 102206 (CN)**

(72) Inventors:
- **XING, Wenlong**
**Beijing 102206 (CN)**
- **JIAO, Ti**
**Beijing 102206 (CN)**
- **LI, Shengxue**
**Beijing 102206 (CN)**
- **WANG, Zhihui**
**Beijing 102206 (CN)**

(74) Representative: **Wang, Bo**
**Panovision IP**
**Ebersberger Straße 3**
**85570 Markt Schwaben (DE)**

# (54) DIPHENOLIC COMPOUND, AND PREPARATION METHOD THEREFOR AND USE THEREOF

(57) The present invention provides a kind of resorcinol-derived compounds shown in formula A, which can be used for preparing drugs for inhibiting hyperpigmentation and a cosmetic and non-therapeutic use for treating hyperpigmentation. The compounds of the present invention have significant effects on inhibiting pigmentation caused by ultraviolet and/or visible light radiation and anti-aging of skin.

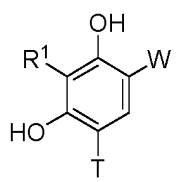

A

,

wherein W is

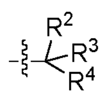

,



EP 4 520 749 A1

and T is H,

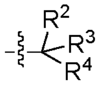
or
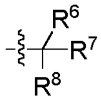
.

$R^1$ to $R^8$ are defined in the description.

(52) Cooperative Patent Classification (CPC): (Cont.)
**C07D 223/10**

C-Sets
**C07C 37/11, C07C 39/15**

## Description

## TECHNICAL FIELD

The present invention relates to a kind of diphenol compounds, the preparation methods thereof, pharmaceutical compositions or cosmetic compositions comprising the compounds, and the use thereof in pharmaceutical application and cosmetics, particularly the use in inhibiting pigmentation and treating hyperpigmentation.

## BACKGROUND

Melanin, which exists in melanocytes and is produced by catalytic conversion of tyrosine by tyrosinase, is a pigment responsible for skin coloring. Excessive production of the melanin can cause hyperpigmentation of the skin due to excessive sun exposure, hormonal imbalance, melasma, diseases, medicated injuries, scars, age spots caused by aging, and other factors.

Human skin colors are mainly determined by the amount of the melanin in a basal layer of the skin. The melanin is usually brown to black, is formed in the melanin-forming cells of the skin, and is transferred to a stratum corneum of the skin to provide colors for the skin or hair. In mammals, the brown-black eumelanin is formed mainly by hydroxy-replaced aromatic amino acids such as L-tyrosine and L-DOPA. In addition, the yellow to red pseudomelanin is formed by sulfur-containing molecules. Starting with L-tyrosine, L-3, 4-dihydroxyphenylalanine (L-DOPA) is formed by tyrosinase and converted to dopachrome by tyrosinase, which is finally oxidized to form melanin through a series of catalytic steps by various enzymes.

When too much melanin is concentrated in one region or part of the skin, the skin appears as hyperpigmentation. Hyperpigmentation may also occur due to exposure to sunlight or due to different inflammatory stimuli. Production of increased melanin is often accompanied by manifestations such as black spots, melasma or solar lentigines (age spots), freckles and pigmented keratoses. Black spot is a general term for describing dull skin. Melasma is often used to describe skin discoloration caused by hormones. These hormonal changes are usually due to pregnancy, contraceptive drugs, or estrogen replacement therapy. Solar lentigines are dark spots on the skin caused by sunlight. These spots are common in adults with a long history of unprotected sun exposure. Although skin lesions such as scars, cuts or rashes can also cause hyperpigmentation, the most common cause of darkened regions, brown spots, or discolored regions of the skin is unprotected sun exposure.

The prior art discloses methods for treating hyperpigmentation through the use of skin brightening agents. Common representative compounds of the skin brightening agents include hydroquinone and vitamin C. One effective way of these agents is to brighten the skin by inhibiting tyrosinase which is involved in melanin production. The most popular ingredients used for skin brightening, skin bleaching or skin whitening include hydroquinone, arbutin, kojic acid, licorice extract, niacinamide, etc. Hydroquinone is one of the most effective decolorants. Although the efficacy has been proven, hydroquinone began to be removed from cosmetics or the use in cosmetics was restricted in the early 2000s due to the potential dermatological and systemic side effects. Arbutin (a natural product extracted from plants) is a glycosylated derivative of hydroquinone and is a potent tyrosinase inhibitor that is more stable and less toxic than hydroquinone. However, despite the great potential, arbutin is still unstable and undergoes hydrolysis under different conditions, resulting in the release of hydroquinone.

The development of safer and more effective compounds that alleviate skin pigmentation has long been a goal. By studying the action mechanism of tyrosinase and simulating an intermediate product L-DOPA in the formation process of melanin, a resorcinol tyrosinase inhibitor is developed, which has received more and more attention from researchers related to skin whitening. This series of resorcinol derivatives, such as 4-butylresorcinol, 4-hexylresorcinol, dimethoxytolyl-4-propyl resorcinol (WO2012/129260), phenylethyl resorcinol (WO2018/001485) and isobutylamido thiazolyl resorcinol (WO2015/090850), have been proved to have significant effects on skin pigmentation. These results indicate that resorcinol compounds have good potential for inhibiting tyrosinase activity. Therefore, the development of novel compounds with resorcinol structure and the exploration of the biological functions as a tyrosinase inhibitor are of great significance for the development of novel and efficient tyrosinase inhibitors, and thus the development of novel compound compositions that treats or improves skin hyperpigmentation.

## SUMMARY

After research, the inventor develops a new kind of resorcinol compounds that can be used as a tyrosinase inhibitor, especially used for cosmetic or pharmaceutical application, more specifically used as a decolorizing agent, a whitening agent, a bleach or a brightening agent, and used for the treatment of pigmentation disorders or for the alleviation or improvement of hyperpigmentation for cosmetic uses.

Particularly, the present invention provides a kind of resorcinol compounds of formula A that can affect

pigmentation of skin regions caused by sunlight, especially ultraviolet and visible light, and particularly can prevent, treat and/or alleviate pigmentation of skin regions or part of skin. The structure of the compounds of formula A is as follows:

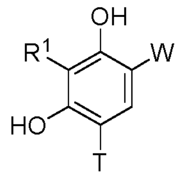

A

,

wherein W is

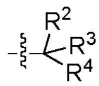

,

Tis H,

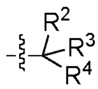

or

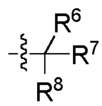

;

wherein $R^1$ is selected from H, optionally substituted alkyl and optionally substituted aryl alkyl;
$R^2$ and $R^6$ can be identical or different, and are independently selected from optionally substituted aryl and heteroaryl, optionally substituted alkyl, optionally substituted amide, and optionally substituted ester respectively; $R^3$, $R^4$, $R^7$ and $R^8$ can be identical or different, and are independently selected from hydrogen, optionally substituted alkyl, optionally substituted cycloalkyl, and optionally substituted amide respectively; $R^3$ and $R^4$ can be connected with carbon atoms jointly connected to form a ring; $R^7$ and $R^8$ can be connected with carbon atoms jointly connected to form a ring; or cosmetically or pharmaceutically acceptable salt, stereoisomers or a mixture of stereoisomers of any proportion, particularly enantiomers or a mixture of enantiomers, and more particularly a racemic mixture.

Further, the compound A is a compound of formula (I),

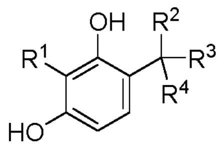

(I),

wherein $R^1$ to $R^4$ are defined as above.
Further, the compound A is a compound of formula (Ia),

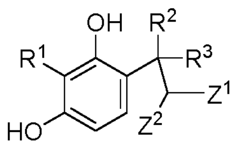

(Ia)

,

wherein $R^1$ to $R^3$ are defined as above; $Z^1$ and $Z^2$ can be identical or different, and are independently selected from hydrogen, optionally substituted alkyl, optionally substituted cycloalkyl and optionally substituted amide respectively; $Z^1$ and $Z^2$ can be connected with carbon atoms jointly connected to form a ring; and the ring can be substituted.

Further, the compound A is a compound of formula (V),

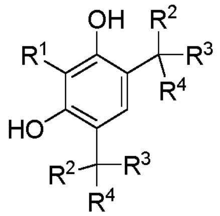

(V),

wherein $R^1$ to $R^4$ are defined as above.

Further, the compound A is a compound of formula (Va),

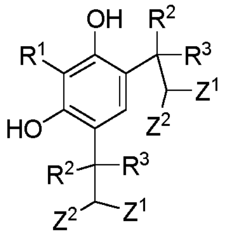

(Va),

wherein $R^1$ to $R^3$ are defined as above; $Z^1$ and $Z^2$ can be identical or different, and are independently selected from hydrogen, optionally substituted alkyl, optionally substituted cycloalkyl, and optionally substituted amide respectively; $Z^1$ and $Z^2$ can be connected with carbon atoms jointly connected to form a ring; and the ring can be substituted.

Further, the compound A is a compound of formula (VI),

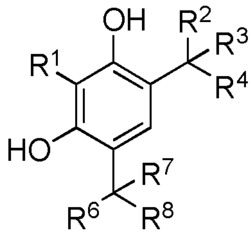

(VI),

wherein $R^1$ to $R^4$ and $R^6$ to $R^8$ are defined as above.

Further, the compound A is (VIa),

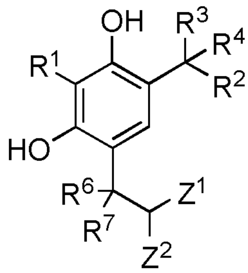

(VIa),

wherein $R^1$ to $R^4$ and $R^6$ to $R^7$ are defined as above; $Z^1$ and $Z^2$ can be identical or different, and are independently selected from hydrogen, optionally substituted alkyl, optionally substituted cycloalkyl, and optionally substituted amide respec-

tively; $Z^1$ and $Z^2$ can be connected with carbon atoms jointly connected to form a ring; and the ring can be substituted.

The present invention further provides preparation methods of the compound A.

The present invention further provides pharmaceutical composition comprising the compounds of the present invention or pharmaceutically acceptable salts thereof.

The present invention further provides a use of the compounds of the present invention or pharmaceutically acceptable salts thereof in preparation of drugs for treating, preventing and/or alleviating hyperpigmentation.

The present invention further discloses a non-therapeutic and cosmetic use of the compounds of the present invention or salts thereof in treating, preventing and/or alleviating hyperpigmentation.

The present invention further discloses a cosmetic composition comprising the compounds of the present invention or salts thereof.

## DETAILED DESCRIPTION

To make a purpose, a technical solution and advantages of embodiments of the present invention more clear, the technical solution in the embodiments of the present invention will be clearly and fully described below. Apparently, the described embodiments are merely part of the embodiments of the present invention, not all of the embodiments. Based on the embodiments in the present invention, all other embodiments obtained by those ordinary skilled in the art without contributing creative labor will belong to the protection scope of the present invention. Unless expressly stated otherwise, throughout the description and claims, the term "comprise" or variations thereof such as "comprising" or "comprises" will be understood to comprise the stated ingredient or steps, not to exclude other material ingredients or steps.

In addition, to better explain the present invention, numerous specific details are given in the specific implementation modes below.

Those skilled in the art should understand that the present invention can be implemented without certain specific details. In some embodiments, raw materials, methods, means, etc. well known to those skilled in the art are not described in detail in order to highlight the subject matter of the present invention.

The endpoints and any values of the ranges disclosed herein are not limited to the exact ranges or values, and these ranges or values shall be understood to contain values close to these ranges or values. For numeric ranges, the endpoint values of each range, the endpoint values of each range and individual point values, and the individual point values can be mutually combined to obtain one or more new numeric ranges, and these numeric ranges shall be deemed to be specifically disclosed herein.

In the present invention, "hyperpigmentation" means hyperpigmentation caused by sunlight, especially visible light.

In the present invention, "cosmetically or pharmaceutically acceptable" means capable of preparation of a cosmetic or pharmaceutical composition and generally non-toxic, safe and acceptable for pharmaceutical and cosmetic uses.

In the present invention, the "cosmetically or pharmaceutically acceptable salt" is a cosmetically or pharmaceutically acceptable salt defined herein and having the medicinal and cosmetic properties and activity of the original compound. The salt can be: (1) acid addition salt formed with inorganic acid such as hydrochloric acid, hydrobromic acid, sulfuric acid, nitric acid, phosphoric acid, etc.; or acid addition salt formed with organic acid such as acetic acid, benzenesulfonic acid, benzoic acid, camphor sulfonic acid, citric acid, ethanesulfonic acid, fumaric acid, glucoheptose acid, gluconic acid, glutamic acid, glycolic acid, hydroxy-naphthoic acid, 2-hydroxy-ethanesulfonic acid, lactic acid, maleic acid, malic acid, mandelic acid, mesylic acid, muconic acid, 2-naphthalenesulfonic acid, propionic acid, salicylic acid, succinic acid, dibenzoyl-L-tartaric acid, tartaric acid, p-toluenesulfonic acid, trimethylacetic acid, trifluoroacetic acid, etc., but not limited to this, and (2) a salt formed when acid protons present in a parent compound are substituted by metal ions such as alkali metal ions (such as $Na^+$, $K^+$ or $Li^+$), alkaline earth metal ions (such as $Ca^{2+}$ or $Mg^{2+}$), or aluminum ions; or a salt formed when acid protons present in the parent compound are coordinated with an organic or inorganic base. Acceptable organic bases comprise diethanolamine, ethanolamine, N-methylglucosamine, triethanolamine, trometamol, etc., but not limited to this. Acceptable inorganic bases comprise aluminum hydroxide, calcium hydroxide, potassium hydroxide, sodium carbonate and sodium hydroxide, but not limited to this.

The term "stereoisomer" used in the present invention refers to a configurational stereoisomer, which comprises a geometric isomer, an optical isomer, and a conformational isomer.

The geometric isomer (also known as E/Z isomer or cis-trans isomer) results from different positions of substituents on C=C double bonds, which can have a Z or E configuration, also known as a cis-trans configuration.

The optical isomer results from substituents or lone pair electrons on atoms (such as carbon atoms or sulfur atoms) that comprise four different substituents (potentially comprising lone pair electrons) at different positions in space. Thus, the atom represents a chiral or asymmetric center. Therefore, optical isomers that are not mirror images of each other are called "diastereoisomers", while optical isomers that are non-overlapping mirror images of each other are called "enantiomers".

An equimolar mixture of two enantiomers of the chiral compound is called a racemic mixture.

Halogens in the present invention represent fluorine, chlorine, bromine and iodine in all cases.

The term "alkyl" used herein (and in other groups comprising alkyl, such as the alkyl structural portion of alkoxy, and the alkyl portion of aryl alkyl) represents a linear or branched alkyl having usually 1-20 carbon atoms, often 1-6 carbon atoms, preferably 1-4 carbon atoms, and especially 1-3 carbon atoms in each case. Examples of $C_1$-$C_4$ alkyl are methyl, ethyl, n-propyl, isopropyl, n-butyl, 1-methylpropyl (sec-butyl), 2-methylpropyl (isobutyl) and 1,1-dimethylethyl (tert-butyl). In addition to the groups mentioned by $C_1$-$C_4$ alkyl, examples of $C_1$-$C_6$ alkyl are n-amyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylamyl, 2-methylamyl, 3-methylamyl, 4-methylamyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethyl butyl, 2-ethyl butyl, 1,1,2-trimethylpropyl, 1,2,2-trimethylpropyl, 1-ethyl-1-methylpropyl or 1-ethyl-2-methylpropyl. In addition to the groups mentioned by $C_1$-$C_6$ alkyl, examples of $C_1$-$C_{10}$ alkyl comprise n-heptyl, 1-methylhexyl, 2-methylhexyl, 3-methylhexyl, 1-ethylhexyl, 2-ethylhexyl, 1,2-dimethylhexyl, 1-propylamyl, 2-propylamyl, nonyl, decyl, 2-propylheptyl and 3-propylheptyl, but not limited to this. Alkyl can be substituted by halogen, cyano, nitro, aryl, cycloalkyl, heterocyclyl, acylamino, ester, etc., but not limited to this.

In the present invention, "aryl" refers to a monovalent monocyclic or bicyclic aromatic hydrocarbon group of 6 to 10 annular atoms, such as phenyl or naphthyl, especially naphthyl, but not limited to this. Aryl can be substituted by alkyl, halogen, cyano, nitro, cycloalkyl, heterocyclyl, acylamino, ester, etc. , but not limited to this.

In the present invention, "ring" refers to "cycloalkyl" or "heterocyclyl", and "cycloalkyl" refers to a monocyclic univalent hydrocarbyl of three to six carbon atoms, which can be saturated or comprises a double-bond. Cycloalkyl may not be substituted or substituted by one or two substituents independently selected from alkyl, halogen, alkoxyl, hydroxyl or cyano, but not limited to this. Examples comprise, but are not limited to, cyclopropyl, cyclobutyl, cycloamyl, cyclohexyl, 1-cyanocycloprop-1-yl, 1-cyanomethylcycloprop-1-yl, 3-fluorocyclohexyl, etc., but not limited to this. When cycloalkyl comprises a double-bond, it can be called cycloalkenyl herein. "Heterocyclyl" refers to a saturated or unsaturated monovalent monocyclic group of 4 to 8 annular atoms, wherein one or two annular atoms are heteroatoms selected from N, O or $S(O)_p$, wherein p is an integer from 0 to 2 and the remaining annular atoms are C. In addition, one or two annular carbon atoms in a heterocyclic ring can be optionally substituted by a -CO-group. More specifically, the term heterocyclyl comprises, but is not limited to, azacyclobutanyl, oxacyclobutanyl, pyrrolidinyl, piperidyl, homopiperidyl, 2-oxopyrrolidinyl, 2-oxopiperidyl, morpholinyl, piperazino, tetrahydropyranyl, thiomorpholinyl, etc. When cycloalkyl, heterocyclyl, etc. are substituted, they can be substituted by alkyl, halogen, cyano, nitro, aryl, acylamino, ester, etc., but not limited to this.

The term "optionally substituted" means that the related group may or may not be substituted by a substituent.

When a group is substituted, the substituents can be alkyl, halogen, cyano, nitro, cycloalkyl, heterocyclyl, acylamino, ester, alkoxyl and halogen, but not limited to this. For example, when alkyl is substituted by a halogen, a haloalkyl is formed, but not limited to this.

"Alkoxy" refers to an -OR group, wherein R is the alkyl as defined above, such as methoxy, ethoxy, propoxy, or 2-propoxy, n-butoxy, isobutoxy, or tert-butoxy, etc., but not limited to this.

"Amide" in the present invention refers to a group comprising "-NHCO-" or "

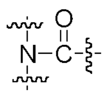

",

and the amide group is, for example, $-NHCOCH_3$, -NHCOH, $-NHCOCH_2CH_3$, $-NHCOCH_2CH_2CH_3$, - $NHCOCH(CH_3)_2$, $-NHCOCH(CH_2)_2$, $-N(COCH_3)_2$, $-CONH_2$, $-CON(CH_3)_2$, $-CONHCH_3$, $-CONHCH_2CH_3$, $-CON(CH_2CH_3)_2$, $-CONHCH(CH_3)_2$, $-CONHCH_2CH_2CH_3$, $-CONHCH_2CH_2CH_2CH_3$, phthaloyl, succinimido, glutarimido, maleimido,

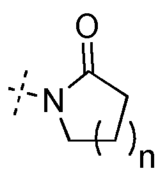, and 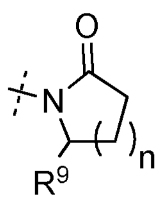,

wherein $R^9$ is selected from optionally substituted alkyl or aryl, carboxyl or a salt thereof, cyano, optionally substituted amide and optionally substituted ester; n is selected from a natural number from 1 to 6; the ester is preferably $-COOCH_3$, $-COOCH_2CH_3$, $-COOCH(CH_3)_2$, $-COOCH_2CH_2CH_3$ and $-COOCH_2CH_2CH_2CH_3$.
n is a natural number from 1 to 6, preferably 1, 2, and 3, but not limited to this.

"Ester" in the present invention refers to a group comprising "-COO-", and the ester is, for example, $-COOCH_3$, $-COOCH_2CH_3$, $-COOCH(CH_3)_2$, $-COOCH_2CH_2CH_3$ and $-COOCH_2CH_2CH_2CH_3$, but not limited to this.

"Heteraryl" in the present invention covers: 5 to 10-membered aromatic single rings and aromatic fused rings, wherein the aromatic single ring comprises one or more (e.g., 1 to 4, or 1 to 3 in some implementation solutions) heteroatoms selected from N, O and S and the remaining atoms are carbon; the aromatic fused ring comprises one or more (e.g., 1 to 4, or 1 to 3 in some implementation solutions) heteroatoms selected from N, O and S and the remaining annular atoms are carbon, and wherein at least one heteroatom is present in the aromatic ring. For example, heteroaryl comprises 5 to 10-membered heterocyclic alkyl aromatic rings gelled with 5 to 10-membered cycloalkyl or heterocyclic alkyl rings. For the fusion of the double-ring heteroaromatic ring system in which only one ring comprises one or more heteroatoms, the joining points may be on any ring. When the total number of S and O atoms in a heteroaryl group exceeds 1, these heteroatoms are not adjacent to each other. In some implementation solutions, the total number of S and O atoms in the heteroaryl group does not exceed 2. In some implementation solutions, the total number of S and O atoms in the aromatic heterocyclic ring does not exceed 1. Examples of the heteroaryl group comprise, but are not limited to, (self-defined as 1-digit connection location number) 2-pyridyl, 3-pyridyl, 4-pyridyl, 2,3-pyridazinyl, 3,4-pyridazinyl, 2,4-pyrimidinyl, 3,5-pyrimidinyl, 2,3-pyrazolinyl, 2,4-imidazolinyl, isooxazolinyl, oxazolinyl, thiazolinyl, thiadiazolinyl, tetraazolyl, thiophenyl, benzothiophenyl, furanyl, benzofuranyl, benzimidazolinyl, indolinyl, pyrazinyl, triazolinyl, quinolinyl, pyrazolyl and 5,6,7,8-tetrahydroisoquinolyl. A divalent group derived from the removal of a hydrogen atom from a free-valent carbon in a monovalent heteraryl group whose name ends with "yl" is named by adding "sub" to the name of the corresponding monovalent group. For example, a pyridyl group with two joining points is a pyridylidene. Heteraryl does not cover or overlap with aryl, cycloalkyl or heterocyclic alkyl, as defined herein.

"Aryl alkyl" refers to a residue in which the aryl part is connected to the parent structure by an alkyl residue. Examples comprise benzyl, phenylethyl, phenyl vinyl, phenyl allyl, etc. "Heterarylalkyl" refers to residues in which the heteraryl part is connected to the parent structure by an alkyl residue. Examples comprise furyl methyl, pyridinyl methyl, pyrimidinyl ethyl, etc.

The following description of the variables of the compound of formula (A) and the preferred implementation solutions of the variables, the features of the uses and methods of the present invention, and the features of the compositions of the present invention are valid either on their own or in preferred combinations with each other.

In general, the present invention provides a kind of resorcinol compounds of formula A,

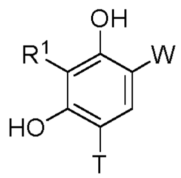

A

,

wherein W is

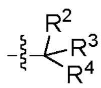

,

T is H,

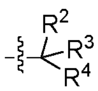

or

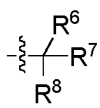

;

wherein $R^1$ is selected from H, optionally substituted alkyl and optionally substituted aryl alkyl;

$R^2$ and $R^6$ can be identical or different, and are independently selected from optionally substituted aryl, optionally substituted heteroaryl, optionally substituted alkyl, optionally substituted amide and optionally substituted ester respectively; $R^3$, $R^4$, $R^7$ and $R^8$ can be identical or different, and are independently selected from hydrogen, optionally

substituted alkyl, optionally substituted cycloalkyl and optionally substituted amide respectively; $R^3$ and $R^4$ can be connected to carbon atoms jointly connected to form a ring; $R^7$ and $R^8$ can be connected to carbon atoms jointly connected to form a ring, wherein the amide group is preferably $-NHCOCH_3$, $-NHCOH$, $-NHCOCH_2CH_3$, $-NHCOCH_2CH_2CH_3$, $-NHCOCH(CH_3)_2$, - $NHCOCH(CH_2)_2$, $-N(COCH_3)_2$, $-CONH_2$, $-CON(CH_3)_2$, $-CONHCH_3$, $-CONHCH_2CH_3$, $-CON(CH_2CH_3)_2$, - $CONHCH(CH_3)_2$, $-CONHCH_2CH_2CH_3$, $-CONHCH_2CH_2CH_2CH_3$, phthaloyl, succinimido, glutarimido, maleimido,

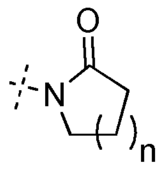 and 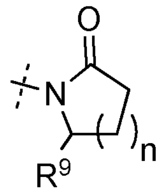,

wherein $R^9$ is selected from optionally substituted alkyl or optionally substituted aryl, carboxyl or a salt thereof, cyano, optionally substituted amide and optionally substituted ester; n is selected from a natural number from 1 to 6; and the ester is preferably one of $-COOCH_3$, $-COOCH_2CH_3$, $-COOCH(CH_3)_2$, $-COOCH_2CH_2CH_3$ and $-COOCH_2CH_2CH_2CH_3$;

or cosmetically or pharmaceutically acceptable salt, stereoisomers or a mixture of stereoisomers of any proportion, particularly enantiomers or a mixture of enantiomers, and more particularly a racemic mixture.

Further, the present invention provides a kind of compounds of formula A which is formula (I) or a salt thereof:

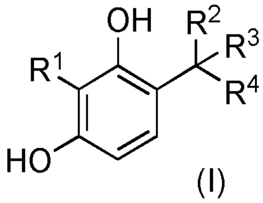

(I),

wherein $R^1$ is selected from H, optionally substituted alkyl and optionally substituted aryl alkyl;

$R^2$ is selected from optionally substituted aryl, optionally substituted alkyl, optionally substituted amide, optionally substituted ester, optionally substituted five to ten-membered heteroaryl (the heteroaryl comprises 1 to 4 heteroatoms independently selected from nitrogen, oxygen or sulfur), preferably optionally substituted alkyl,

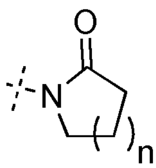 and 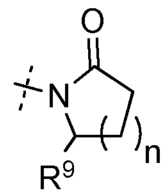,

wherein $R^9$ is selected from optionally substituted alkyl or aryl, carboxyl or a salt thereof, cyano, optionally substituted amide and optionally substituted ester; n is selected from a natural number from 1 to 6, preferably 1, 2 and 3; $R^3$ and $R^4$ can be identical or different, and are independently selected from hydrogen, optionally substituted alkyl, optionally substituted cycloalkyl and optionally substituted amide respectively; and $R^3$ and $R^4$ can be connected to carbon atoms jointly connected to form a ring.

Further, in some implementation modes of formula (I), $R^1$ is selected from H and $C_1$-$C_6$ alkyl, particularly preferably H and methyl.

Further, in some implementation modes of formula (I), $R^2$ is selected from

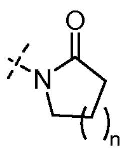,

wherein n is 1, 2 or 3.

Further, in some other implementation modes of formula (I), $R^2$ is selected from

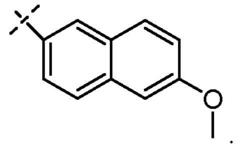

Further, in some implementation modes of formula (I), $R^2$ is selected from phenyl.

In some implementation modes of formula (I), preferably, $R^3$ is selected from H and $C_1$-$C_6$ alkyl, particularly preferably one of H, methyl, ethyl, propyl and butyl;

In some implementation modes of formula (I), preferably, $R^4$ is selected from H and $C_1$-$C_6$ alkyl, particularly preferably one of H, methyl, ethyl, propyl and butyl.

Further, the present invention preferably provides formula (I) compounds, such as compounds of the following formulas (I-1), (I-2) and (I-3):

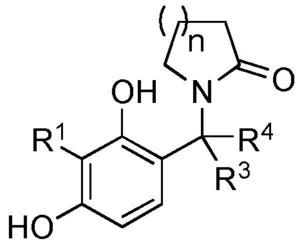

(I-1) ,

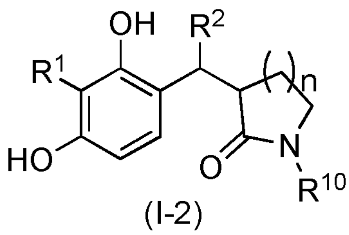

(I-2)

and

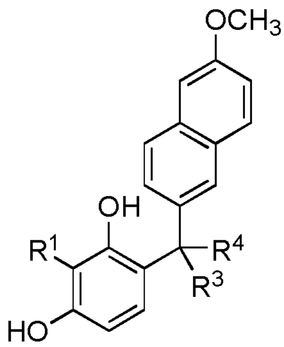

(I-3) ,

wherein $R^1$ is selected from H and $C_1$-$C_6$ alkyl, particularly preferably H and methyl; and $R^2$ is selected from phenyl. In some implementation modes, preferably, $R^3$ is selected from H and $C_1$-$C_6$ alkyl, particularly preferably one of H, methyl, ethyl, propyl and butyl; $R^4$ is selected from H and $C_1$-$C_6$ alkyl, particularly preferably H, methyl, ethyl, propyl and butyl; $R^{10}$ is selected from $C_1$-$C_{18}$ alkyl, preferably n-dodecyl, n-tetradecyl and n-cetyl; and n is selected from 1, 2 and 3.

Further, a compound of formula A provided by the present invention is preferably a compound of formula (Ia),

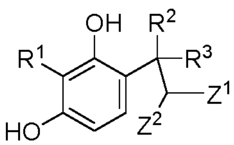

(Ia) ,

wherein $R^1$ is selected from H, optionally substituted alkyl and optionally substituted aryl alkyl; $R^2$ is selected from optionally substituted aryl, optionally substituted five to ten-membered heteroaryl (the five to ten-membered heteroaryl comprises one to four heteroatoms independently selected from nitrogen, oxygen, or sulfur), optionally substituted alkyl and optionally substituted amide; $R^3$ is selected from hydrogen, optionally substituted alkyl, optionally substituted cycloalkyl and optionally substituted amide; $Z^1$ and $Z^2$ can be identical or different, and are independently selected from hydrogen, optionally substituted alkyl, optionally substituted cycloalkyl and optionally substituted amide; $Z^1$ and $Z^2$ can be connected with carbon atoms jointly connected to form a ring; and the ring can be substituted by a substituent.

Further, in the compound of formula (Ia), $R^1$ is selected from H and $C_1$-$C_6$ alkyl, particularly preferably H and methyl.

Further, in some implementation modes of the compound of formula (Ia), $R^2$ is selected from one of

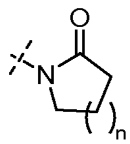

(wherein n is a natural number from 1 to 6),

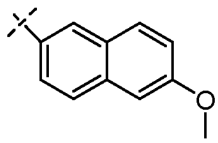

and phenyl.

Further, the compounds of formula (Ia) in the present invention are preferably the following compounds of formulas Ia-1 and Ia-2:

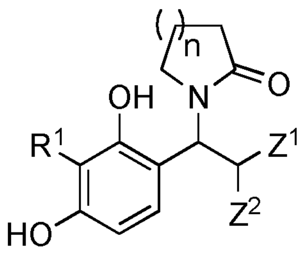

(Ia-1) and

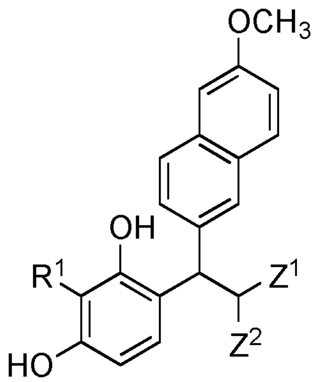

(Ia-2) ,

wherein $R^1$ is selected from H and $C_1$-$C_6$ alkyl, preferably H and methyl;

$Z^1$ and $Z^2$ are independently selected from H and $C_1$-$C_6$ alkyl respectively, preferably one of H, methyl, ethyl, propyl and butyl; or $Z^1$ and $Z^2$ together with connected carbon atoms form $C_3$-$C_6$ cycloalkyl and 3-6-membered heterocyclyl; $C_3$-$C_6$ cycloalkyl is preferably cyclopropyl, cyclobutyl, cycloamyl and cyclohexyl; 3-6-membered heterocyclyl is preferably ethylene oxide, azacyclobutane, oxacyclobutane, pyrrolidinyl, piperidyl, homopiperidyl, 2-oxopyrrolidinyl, 2-oxopiperidyl, morpholinyl, piperazinyl, tetrahydropyranyl and thiomorpholinyl.

n is selected from a natural number from 1 to 6, preferably 1, 2 and 3.

Further, in some implementation solutions, the compound A of the present invention is preferably a compound of formula (V).

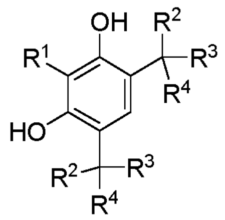

(V),

wherein $R^1$ is selected from H, optionally substituted alkyl and optionally substituted aryl alkyl;

$R^2$ is selected from optionally substituted aryl, optionally substituted alkyl and optionally substituted amide; the amide group is preferably one of $-NHCOCH_3$, -NHCOH, $-NHCOCH_2CH_3$, - $NHCOCH_2CH_2CH_3$, $-NHCOCH(CH_3)_2$, $-NHCOCH(CH_2)_2$, $-N(COCH_3)_2$, $-CONH_2$, $-CON(CH_3)_2$, - $CONHCH_3$, $-CONHCH_2CH_3$, $-CON(CH_2CH_3)_2$, $-CONHCH(CH_3)_2$, $-CONHCH_2CH_2CH_3$, - $CONHCH_2CH_2CH_2CH_3$, phthaloyl, succinimido, glutarimido, maleimido,

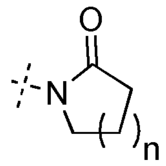

and

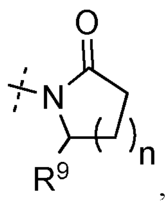

,

wherein $R^9$ is selected from optionally substituted alkyl or aryl, carboxyl or a salt thereof, optionally substituted ester (the ester is preferably one of $-COOCH_3$, $-COOCH_2CH_3$, $-COOCH(CH_3)_2$, - $COOCH_2CH_2CH_3$ and $-COOCH_2CH_2CH_2CH_3$), cyano and optionally substituted amide; n is a natural number from 1 to 6; $R^3$ and $R^4$ can be identical or different, and are independently selected from hydrogen, optionally substituted alkyl, optionally substituted cycloalkyl and optionally substituted amide; and $R^3$ and $R^4$ can be connected with carbon atoms jointly connected to form a ring.

Further, in some implementation solutions, the compound of formula (V) is a compound of formula (Va),

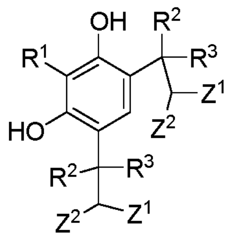

(Va),

wherein $R^1$ is selected from H, optionally substituted alkyl and optionally substituted aryl alkyl;

$R^2$ is selected from optionally substituted aryl, optionally substituted alkyl and optionally substituted amide; the amide group is preferably one of $-NHCOCH_3$, -NHCOH, $-NHCOCH_2CH_3$, - $NHCOCH_2CH_2CH_3$, $-NHCOCH(CH_3)_2$, $-NHCOCH(CH_2)_2$, $-N(COCH_3)_2$, $-CONH_2$, $-CON(CH_3)_2$, - $CONHCH_3$, $-CONHCH_2CH_3$, $-CON(CH_2CH_3)_2$, $-CONHCH(CH_3)_2$, $-CONHCH_2CH_2CH_3$, - $CONHCH_2CH_2CH_2CH_3$, phthaloyl, succinimido, glutarimido, maleimido,

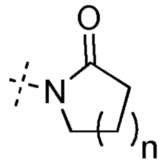

and

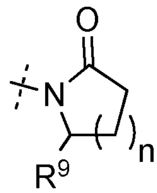

,

wherein $R^9$ is selected from optionally substituted alkyl or aryl, carboxyl or a salt thereof, optionally substituted ester (the ester is preferably one of $-COOCH_3$, $-COOCH_2CH_3$, $-COOCH(CH_3)_2$, - $COOCH_2CH_2CH_3$ and $-COOCH_2CH_2CH_2CH_3$), cyano and optionally substituted amide; n is a natural number from 1 to 6; $R^3$ is selected from hydrogen, optionally substituted alkyl, optionally substituted cycloalkyl and optionally substituted amide; $Z^1$ and $Z^2$ can be identical or different, and are independently selected from hydrogen, optionally substituted alkyl, optionally substituted cycloalkyl and optionally substituted amide; $Z^1$ and $Z^2$ can be connected with carbon atoms jointly connected to form a ring; and the ring can be substituted.

Further, in some embodiments of the present invention, the provided compound of formula A is preferably a compound of (VI),

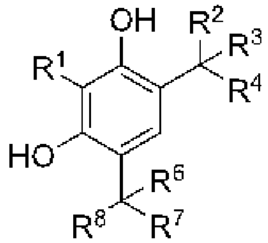

(VI),

wherein $R^1$ is selected from H, optionally substituted alkyl and optionally substituted aryl alkyl; $R^2$ and $R^6$ can be identical or different, and are independently selected from optionally substituted aryl, five to ten-membered heteraryl (the five to ten-membered heteraryl comprises one to four heteroatoms independently selected from nitrogen, oxygen or sulfur), optionally substituted alkyl, optionally substituted ester and optionally substituted amide, wherein the amide is preferably one of $-NHCOCH_3$, -NHCOH, $-NHCOCH_2CH_3$, $-NHCOCH_2CH_2CH_3$, - $NHCOCH(CH_3)_2$, $-NHCOCH(CH_2)_2$, $-N(COCH_3)_2$, $-CONH_2$, $-CON(CH_3)_2$, $-CONHCH_3$, $-CONHCH_2CH_3$, $-CON(CH_2CH_3)_2$, $-CONHCH(CH_3)_2$, $-CONHCH_2CH_2CH_3$, $-CONHCH_2CH_2CH_2CH_3$, phthaloyl, succinimido, glutarimido, maleimido,

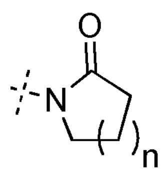 and 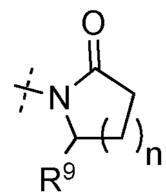 ,

wherein $R^9$ is selected from optionally substituted alkyl or aryl, carboxyl or a salt thereof, optionally substituted ester (the ester is preferably one of $-COOCH_3$, $-COOCH_2CH_3$, $-COOCH(CH_3)_2$, $-COOCH_2CH_2CH_3$ and - $COOCH_2CH_2CH_2CH_3$), cyano and optionally substituted amide; n is a natural number from 1 to 6; $R^3$, $R^4$, $R^7$ and $R^8$ can be identical or different, and are independently selected from hydrogen, optionally substituted alkyl, optionally substituted cycloalkyl and optionally substituted amide respectively; $R^3$ and $R^4$ can be connected to carbon atoms jointly connected to form a ring; $R^7$ and $R^8$ can be connected to carbon atoms jointly connected to form a ring; and the ring can be substituted.

Further, in some implementation modes of formula (VI), $R^2$ and $R^6$ are selected from

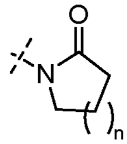,

and n is a natural number from 1-6.

Further, in some implementation modes of formula (VI), $R^2$ and $R^6$ are selected from phenyl.

Further, formula (VI) of the present invention preferably provides the following compounds of formulas (VI-1) and (VI-2):

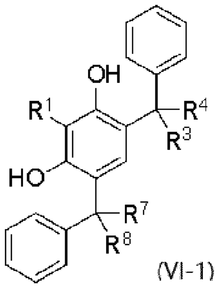

(VI-1)

and

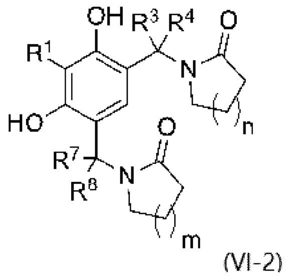

(VI-2),

wherein $R^1$ is selected from H and $C_1$-$C_6$ alkyl, preferably H and methyl; $R^3$ is selected from H and $C_1$-$C_6$ alkyl, preferably one of H, methyl, ethyl, propyl and butyl; $R^4$ is selected from H and $C_1$-$C_6$ alkyl, preferably one of H, methyl, ethyl, propyl and butyl; $R^7$ is selected from H and $C_1$-$C_6$ alkyl, preferably H, methyl, ethyl, propyl and butyl; $R^8$ is selected from H and $C_1$-$C_6$ alkyl, preferably methyl, ethyl, propyl and butyl; n is selected from 1, 2 and 3, and m is selected from 1, 2 and 3.

Further, in some implementation solutions of the present invention, formula (VI) is preferably formula (VIa),

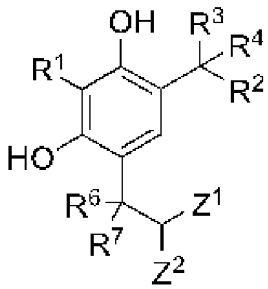

(VIa),

wherein $R^1$ is selected from H, optionally substituted alkyl and optionally substituted aryl alkyl; $R^2$ and $R^6$ can be identical or different, and are independently selected from optionally substituted aryl, five to ten-membered heteraryl (the five to ten-membered heteraryl comprises one to four heteroatoms independently selected from nitrogen, oxygen or sulfur), optionally substituted alkyl, optionally substituted ester and optionally substituted amide, wherein the amide is preferably one of $-NHCOCH_3$, $-NHCOH$, $-NHCOCH_2CH_3$, $-NHCOCH_2CH_2CH_3$, $-NHCOCH(CH_3)_2$, $-NHCOCH(CH_2)_2$, $-N(COCH_3)_2$, $-CONH_2$, $-CON(CH_3)_2$, $-CONHCH_3$, $-CONHCH_2CH_3$, $-CON(CH_2CH_3)_2$, $-CONHCH(CH_3)_2$, $-CONHCH_2CH_2CH_3$, $-CONHCH_2CH_2CH_2CH_3$, phthaloyl, succinimido, glutarimido, maleimido,

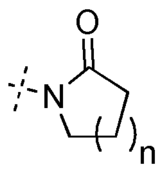 and 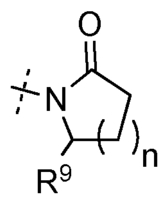,

wherein $R^9$ is selected from optionally substituted alkyl or aryl, carboxyl or a salt thereof, optionally substituted ester (the ester is preferably one of $-COOCH_3$, $-COOCH_2CH_3$, $-COOCH(CH_3)_2$, $-COOCH_2CH_2CH_3$ and - $COOCH_2CH_2CH_2CH_3$), cyano and optionally substituted amide; n is a natural number from 1 to 6; $R^3$, $R^4$ and $R^7$ can be identical or different, and are independently selected from hydrogen, optionally substituted alkyl, optionally substituted cycloalkyl and optionally substituted amide respectively; $R^3$ and $R^4$ can be connected to carbon atoms jointly connected to form a ring; $Z^1$ and $Z^2$ can be identical or different, and are independently selected from hydrogen, optionally substituted alkyl, optionally substituted cycloalkyl and optionally substituted amide respectively; $Z^1$ and $Z^2$ can be connected to carbon atoms jointly connected to form a ring; and the ring can be substituted.

Particularly preferably, the compounds of the present invention are selected from the following compounds:

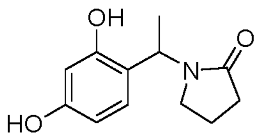

N-(1-(2,4-dihydroxyphenyl) ethyl)-2-pyrrolidone;

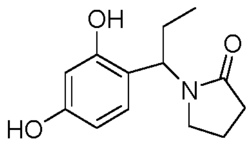

N-(1-(2,4-dihydroxyphenyl) propyl)-2-pyrrolidone;

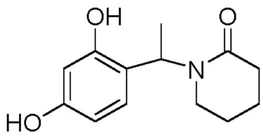

N-(1-(2,4-dihydroxyphenyl) ethyl)-2-piperidone;

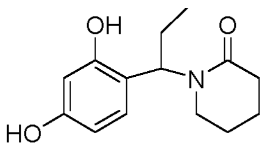

N-(1-(2,4-dihydroxyphenyl) propyl)-2-piperidone;

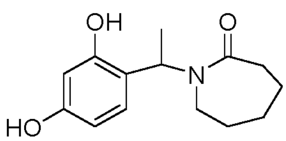

N-(1-(2,4-dihydroxyphenyl) ethyl)-2-azepanone ;

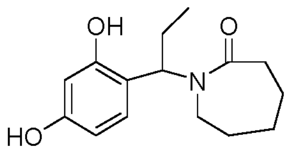

N-(1-(2,4-dihydroxyphenyl) propyl)-2-azepanone;

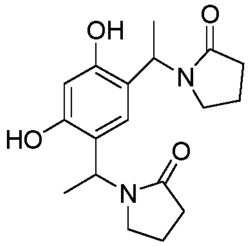

1,1'-((4,6-dihydroxy-1,3-phenylene) bis (ethane-1,1-diyl)) bis (pyrrolidin-2-one);

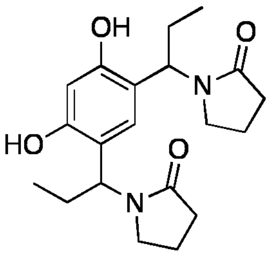

1,1'-((4,6-dihydroxy-1,3-phenylene) bis (propane-1,1-diyl)) bis (pyrrolidin-2-one);

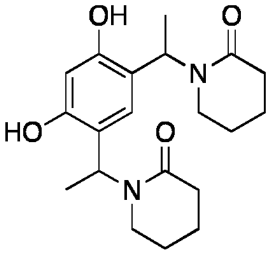

1,1'-((4,6-dihydroxy-1,3- phenylene) bis (ethane-1,1-diyl)) bis (piperidin-2-one);

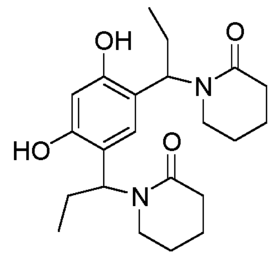

1,1'-((4,6-dihydroxy-1,3- phenylene) bis (propane-1,1-diyl)) bis (piperidin-2-one);

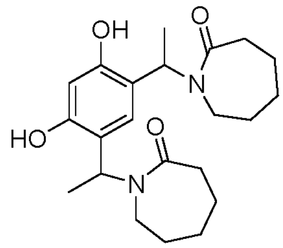

1,1'-((4,6-dihydroxy-1,3- phenylene) bis (ethane-1,1-diyl)) bis (azepan-2-one);

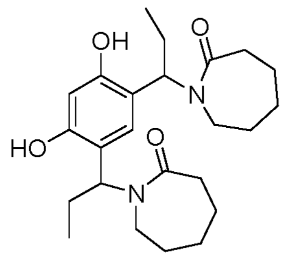

1,1'-((4,6-dihydroxy-1,3- phenylene) bis (propane-1,1-diyl)) bis (azepan-2-one);

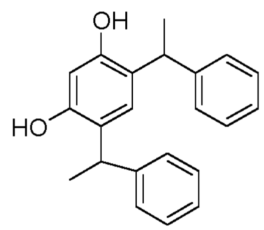

4,6-bis (1-phenylethyl)-1,3-resorcinol.

The present invention further provides a preparation method of the compound of formula (I), with a synthetic route as follows:

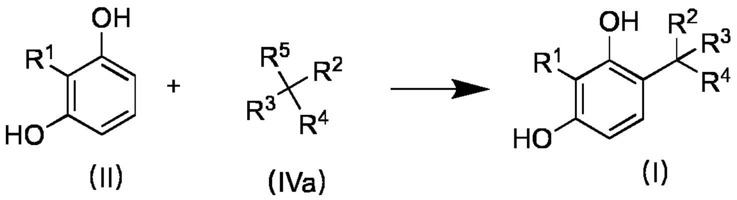

,

wherein $R^1$, $R^2$, $R^3$ and $R^4$ are defined as above, wherein $R^5$ is selected from a leaving group, and the leaving group is selected from one of OH, $H_2O$, OTs, OMs, Cl, Br and I.

In the above reaction, reaction temperature is 40-110°C, preferably 70-90°C.

In the above reaction, a feeding molar ratio of formula II and formula IVa in the reaction is 1:1-1.5, preferably 1:1.01-1.05.

The above reaction is conducted in a solvent, and the solvent is selected from one or a combination of more of toluene, acetonitrile, dioxane, DMF, DMAc, DMSO, NMP, DMI, ethyl acetate, isopropyl acetate, dichloroethane, etc., preferably toluene.

The above reaction can be conducted under the catalysis of a catalyst, and the catalyst is protic acid or Lewis acid, and is selected from hydrochloric acid, HBr, HI, HF, sulfuric acid, phosphoric acid, oxalic acid, methanesulfonic acid, trifluoromethanesulfonic acid, boric acid, benzene sulfonic acid, p-toluenesulfonic acid, etc., preferably p-toluenesulfonic acid.

The present invention further provides a preparation method of the compound of formula (Ia), with a synthetic route as follows:

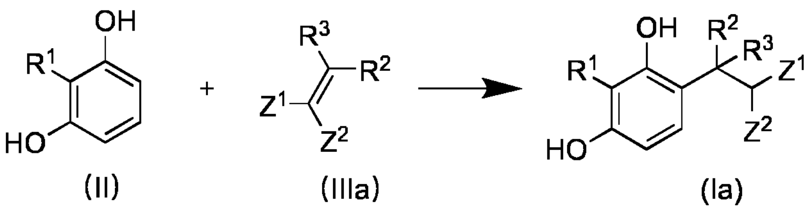

,

wherein $R^1$, $R^2$ and $R^3$ are defined as above; $Z^1$ and $Z^2$ can be identical or different, and are independently selected from hydrogen, optionally substituted alkyl, optionally substituted cycloalkyl and optionally substituted amide respectively; and $Z^1$ and $Z^2$ can be connected to carbon atoms jointly connected to form a ring.

In the above reaction, reaction temperature is 40-110°C, preferably 70-90°C.

In the above reaction, a feeding molar ratio of formula II and formula IIIa in the reaction is 1:1-1.5, preferably 1:1.01-1.05.

The above reaction is conducted in a solvent, and the solvent is selected from one or a combination of more of toluene, acetonitrile, dioxane, DMF, DMAc, DMSO, NMP, DMI, ethyl acetate, isopropyl acetate, dichloroethane, etc., preferably toluene.

The above reaction can be conducted under the catalysis of a catalyst, and the catalyst is protic acid or Lewis acid, and is selected from hydrochloric acid, HBr, HI, HF, sulfuric acid, phosphoric acid, oxalic acid, methanesulfonic acid, trifluoromethanesulfonic acid, boric acid, benzene sulfonic acid, p-toluenesulfonic acid, etc., preferably p-toluenesulfonic acid.

The present invention further provides a preparation method of the compound of formula (V), with a synthetic route as follows: [0124]

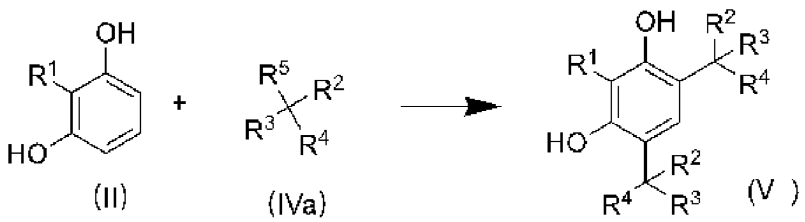

,

wherein $R^1$, $R^2$, $R^3$ and $R^4$ are defined as above, wherein $R^5$ is selected from a leaving group, and the leaving group is

selected from OH, $H_2O$, OTs, OMs, Cl, Br, I, etc.

In the above reaction, reaction temperature is 40-110°C, preferably 70-90°C.

In the above reaction, a feeding molar ratio of formula II and formula IVa in the reaction is 1:2.0-3.0, preferably 1:2.01-2.10.

The above reaction is conducted in a solvent, and the solvent is selected from one or a combination of more of toluene, acetonitrile, dioxane, DMF, DMAc, DMSO, NMP, DMI, ethyl acetate, isopropyl acetate, dichloroethane, etc., preferably toluene.

The above reaction can be conducted under the catalysis of a catalyst, and the catalyst is protic acid or Lewis acid, and is selected from hydrochloric acid, HBr, HI, HF, sulfuric acid, phosphoric acid, oxalic acid, methanesulfonic acid, trifluoromethanesulfonic acid, boric acid, benzene sulfonic acid, p-toluenesulfonic acid, etc., preferably p-toluenesulfonic acid.

The present invention further provides a preparation method of the compound of formula (Va), with a synthetic route as follows:

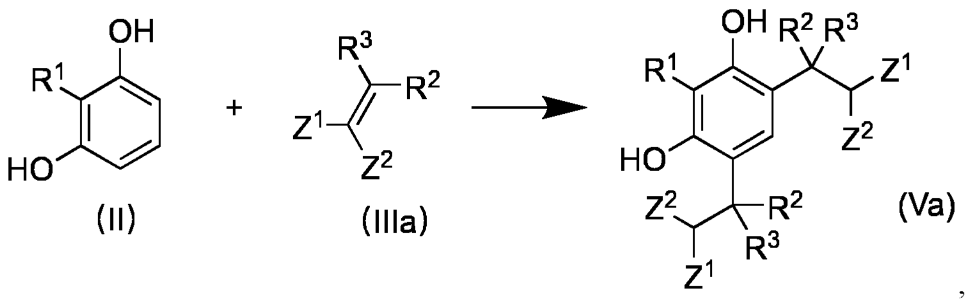

wherein $R^1$, $R^2$ and $R^3$ are defined as above; $Z^1$ and $Z^2$ can be identical or different, and are independently selected from hydrogen, optionally substituted alkyl, optionally substituted cycloalkyl and optionally substituted amide respectively; and $Z^1$ and $Z^2$ can be connected to carbon atoms jointly connected to form a ring.

In the above reaction, reaction temperature is 40-110°C, preferably 70-90°C.

In the above reaction, a feeding molar ratio of formula II and formula IIIa in the reaction is 1:2.0-3.0, preferably 1:2.01-2.10.

The above reaction is conducted in a solvent, and the solvent is selected from one or a combination of more of toluene, acetonitrile, dioxane, DMF, DMAc, DMSO, NMP, DMI, ethyl acetate, isopropyl acetate, dichloroethane, etc., preferably toluene.

The above reaction can be conducted under the catalysis of a catalyst, and the catalyst is protic acid or Lewis acid, and is selected from hydrochloric acid, HBr, HI, HF, sulfuric acid, phosphoric acid, oxalic acid, methanesulfonic acid, trifluoromethanesulfonic acid, boric acid, benzene sulfonic acid, p-toluenesulfonic acid, etc., preferably p-toluenesulfonic acid.

The present invention further provides a preparation method of the compound of formula (VI), with a synthetic route as follows:

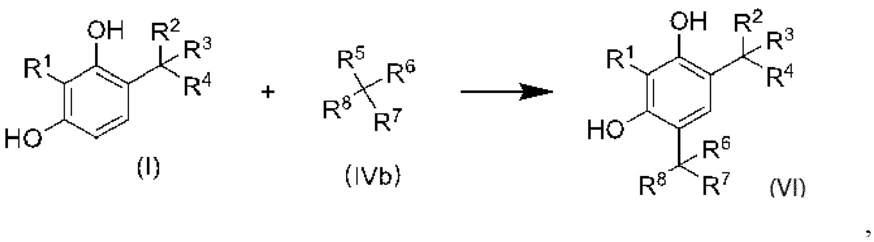

wherein $R^1$, $R^2$, $R^3$, $R^4$, $R^6$, $R^7$ and $R^8$ are defined as above, wherein $R^5$ is selected from a leaving group, and the leaving group is selected from OH, $H_2O$, OTs, OMs, Cl, Br, I, etc.

In the above reaction, reaction temperature is 40-110°C, preferably 70-90°C.

In the above reaction, a feeding molar ratio of formula I and formula IVb in the reaction is 1:1-1.5, preferably 1:1.01-1.05.

The above reaction is conducted in a solvent, and the solvent is selected from one or a combination of more of toluene, acetonitrile, dioxane, DMF, DMAc, DMSO, NMP, DMI, ethyl acetate, isopropyl acetate, dichloroethane, etc., preferably toluene.

The above reaction can be conducted under the catalysis of a catalyst, and the catalyst is protic acid or Lewis

acid, and is selected from hydrochloric acid, HBr, HI, HF, sulfuric acid, phosphoric acid, oxalic acid, methanesulfonic acid, trifluoromethanesulfonic acid, boric acid, benzene sulfonic acid, p-toluenesulfonic acid, etc., preferably p-toluenesulfonic acid.

The present invention further provides a preparation method of the compound of formula (VIa), with a synthetic route as follows:

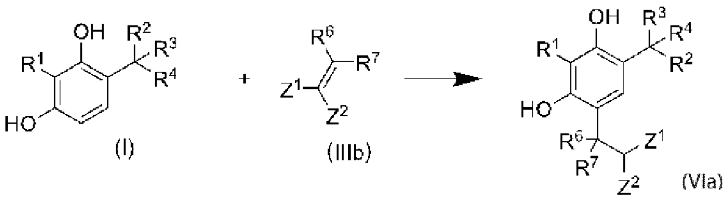

(I) (IIIb) (VIa)

,

wherein $R^1$, $R^2$, $R^3$, $R^4$, $R^6$, $R^7$, $Z^1$ and $Z^2$ are defined as above.

In the above reaction, reaction temperature is 40-110°C, preferably 70-90°C.

In the above reaction, a feeding molar ratio of formula I and formula IIIb in the reaction is 1:1-1.5, preferably 1:1.01-1.05.

The above reaction is conducted in a solvent, and the solvent is selected from one or a combination of more of toluene, acetonitrile, dioxane, DMF, DMAc, DMSO, NMP, DMI, ethyl acetate, isopropyl acetate, dichloroethane, etc., preferably toluene.

The above reaction can be conducted under the catalysis of a catalyst, and the catalyst is protic acid or Lewis acid, and is selected from hydrochloric acid, HBr, HI, HF, sulfuric acid, phosphoric acid, oxalic acid, methanesulfonic acid, trifluoromethanesulfonic acid, boric acid, benzene sulfonic acid, p-toluenesulfonic acid, etc., preferably p-toluenesulfonic acid.

The compounds of the present invention can be used in the application of the preparation of drugs or cosmetics for treating, preventing and/or alleviating hyperpigmentation. The application is particularly local application to skin, such as human skin.

The present invention further provides a cosmetic composition or a pharmaceutical composition comprising the compound of formula (A) in the present invention or a salt thereof.

Further, the cosmetic composition can be prepared into cream, emulsion, ointments, capsules, lotion, foam, gel, dispersants, suspension, spray, essence, mask and any other appropriate form.

Further, the pharmaceutical/cosmetic composition in the present invention can further comprise one or more additives, such as an antioxidant, an emollient, a humectant, a thickener, an aromatic, a preservative, a pigment or a colorant or sunscreen. These additives are conventional to those skilled in the art.

The antioxidant can be used to protect the components of the composition so as to resist an oxidant that is included in the composition or will be in contact with the composition. Examples of the antioxidant comprise ascorbyl palmitate, butylated hydroxyanisole, butylated hydroxytoluene, potassium propyl gallate, octyl gallate, dodecyl gallate, phenyl-$\alpha$-naphthylamine and tocopherols such as $\alpha$-tocopherol.

The emollient is an agent capable of softening and smoothening skin. Examples of the emollient comprise oil and wax (such as microcrystalline wax and polyethylene), triglyceride fatty acids (such as castor oil, cocoa butter, safflower oil, corn oil, olive oil, cod liver oil, almond oil, palm oil and soybean oil), squalene, acetylated monoglyceride, ethoxylated glyceryl ester, fatty acids, alkyl esters of fatty acids, alkenyl esters of fatty acids, fatty alcohol, fatty alcohol ethers, ether-ester, lanum and lanum derivatives, polyol esters, wax esters (such as beewax and vegetable wax), phospholipid and sterol, isopropyl palmitate or stearin, particularly the almond oil or the fatty alcohol (such as cetanol, stearyl alcohol and/or myristyl alcohol).

Siloxane is a particularly preferred emollient. In the present invention, available siloxane comprises but not limited to dimethyl siloxane, methyl cyclosiloxane, phenyl trimethicone, phenyl dimethicone, cetyl dimethicone, stearyl dimethicone, amino-capped dimethylsiloxane, $C_{30-45}$ alkyl dimethylsiloxane, $C_{30-45}$ alkyl methylsiloxane, cetearyl methylsiloxane, dimethicone copolyol, cyclopentylsiloxane, cyclohexylsiloxane or any combination. Particularly, in the present invention, the amino-capped dimethylsiloxane can serve as the emollient.

The humectant is used for increasing and retaining skin moisture. Examples of the humectant comprise propylene glycol, butanediol, polyethylene glycol (PEG) (such as PEG-4 to PEG-32), glycerol (also called glycerin), sorbitol, xylitol, maltitol, mannitol, dextrosan, hyaluronic acid or salts thereof (such as sodium salts or potassium salts), urea, aloe juice, honey, etc.

The thickener is used for increasing viscosity and consistency of the composition. Examples of the thickener comprise a lipid thickener such as cetanol, stearyl alcohol, myristyl alcohol, carnauba wax or a stearic acid, a naturally

derived thickener such as cellulose derivatives, e.g., hydroxyethyl cellulose, guar gum, locust bean gum, xanthan gum or gelatin, a mineral thickener such as silica, bentonite or aluminium magnesium silicate, a synthetic thickener such as carbomer, and an ion thickener such as NaCl.

Examples of the aromatic or perfume comprise peppermint, rose oil, rose water, aloe juice, clove oil, menthol, camphor, eucalyptus oil and any other plant extract. To eliminate certain smell of the composition, a masking agent can be used.

The preservative can be used for preventing the composition from being degraded. Examples of the preservative comprise phenoxyethanol, methyl p-hydroxybenzoate, benzalkonium chloride, benzethonium chloride, propyl p-hydroxybenzoate, benzoic acid, benzyl alcohol and a mixture thereof. Particularly, the preservative can be the phenoxyethanol, methyl p-hydroxybenzoate or a mixture thereof.

The pigment or colorant is used for changing the color of the composition. In addition to the soluble substances, insoluble photoprotective dyes, particularly finely dispersed metallic oxides or salts are applicable to the purpose. For example, to obtain a white composition, titanium dioxide can be selected. In a clarified or transparent composition, sunscreen such as titanium oxide is used, such that the composition is opaque. Particularly appropriate instances of the metallic oxides further comprise zinc oxide, iron, zirconium, silicon, manganese, aluminum and cerium oxides and mixtures thereof. Silicate (talc), barium sulfate or zinc stearate can serve as an instance of the appropriate salt. The oxides and salts are used for skin care and used in the form of a pigment of skin protection lotion and decorative cosmetics. Under the circumstance, the particles should have the mean diameter of less than 100 nm, preferably 5-50 nm, particularly preferably 15-30 nm. The particles can be spherical, but also can be elliptical particles or particles in other shapes rather than spherical. The pigment can be subjected to surface treatment, that is, hydrophilization or hydrophobization. A typical instance is coated titanium dioxide. Under the circumstance, an appropriate hydrophobic coating agent is preferably organosilicone, particularly trialkoxy octylsilane or tripolysiloxane. Micro or nano dyes are preferably used in sunscreen preparations, preferably micronized zinc oxide is used.

In preferred implementation solutions, the photoprotective dye is selected from superfine titanium dioxide, zinc oxide and superfine zinc oxide. When the titanium dioxide serves as the photoprotective dye, it is beneficial that the total amount of the dye is 0.1-10.0 wt% of the preparation. When the zinc oxide serves as the photoprotective dye, it is beneficial that the total amount of the dye is 0.1-10.0 wt% of the preparation. Moreover, when one or more triazine organic pigments are selected, it is beneficial that the total amount of the dye is 0.1-10.0 wt%, based on the total amount of the preparation. In one preferred implementation solution, the pharmaceutical/cosmetic composition according to the present invention further comprises at least one skin brightening agent such as sclareolide.

The present invention further relates to a cosmetic use of the compound of formula (A) according to the present invention, particularly a cosmetic use while serving as a decolorant, a whitener, a bleacher or a brightener, more particularly used for skin, such as human skin.

The present invention further relates to a cosmetic use of the composition of the cosmetic compounds according to the present invention, particularly a cosmetic use while serving as a decolorant, a whitener, a bleacher or a brightener composition, more particularly local application to skin, such as human skin.

The present invention further relates to a use of the compound of formula (A) according to the present invention in preparation of a cosmetic composition. The cosmetic composition is particularly used for decoloring, whitening, bleaching or brightening kin, such as human skin.

The present invention further relates to a use of the compound of formula (A) according to the present invention serving as a decolorant, a whitener, a bleacher or a brightener, more particularly used for skin, such as human skin.

The present invention further relates to a method for decoloring, whitening, bleaching or brightening skin, such as human skin by applying an effective amount of the compound according to the present invention or the cosmetic composition according to the present invention to skin of a person in need.

The present invention further relates to a use of the compound according to the present invention serving as a drug, particularly in treatment of pigmentation diseases, more particularly local application to skin, such as human skin.

The present invention further relates to a use of the pharmaceutical composition according to the present invention, particularly a dermatological composition, serving as a drug, particularly in treatment of pigmentation diseases, more particularly local application to skin, such as human skin.

The present invention further relates to a use of the compound according to the present invention in preparation of a pharmaceutical composition, particularly a dermatological composition, wherein the pharmaceutical composition, particularly the dermatological composition, particularly aims to be used for treating pigmentation diseases, more particularly local application to skin, such as human skin.

The present invention further relates to a use of the compound according to the present invention in treatment of pigmentation diseases, more particularly local application to skin, such as human skin.

The present invention further relates to a method for treating pigmentation diseases of skin such as human skin by applying an effective amount of the compound according to the present invention or the pharmaceutical composition according to the present invention to skin of a person in need.

The pigmentation diseases more particularly refer to hyperpigmentation, comprising lentigo, black spot, freckle and hyperpigmentation after inflammation and hyperpigmentation caused by drugs, chemical substances or sunlight.

The present invention further relates to a use of the compound according to the present invention serving as an antioxidant, particularly inhibiting or decreasing oxidative stress, particularly oxidative stress because of UV, more particularly oxidative stress in skin.

The present invention further relates to a method for inhibiting or decreasing oxidative stress, particularly oxidative stress because of UV, more particularly oxidative stress in skin. The method comprises application, particularly local application of an effective amount of the compound according to the present invention to a person in need.

When the composition in the present invention is applied to human skin, a combination of the compound in the present invention and UV filter substances is well tolerated, thereby avoiding skin reddening, whitening or browning, and avoiding irritation, dry skin or adhesive membrane, not forming wet, scaly, powdered, or even chapped skin. These UV filter substances can be a UV-A filter substance, a UV-B filter substance, a photoprotective dye or a mixture thereof. The UV filter substances refer to, for example, organic substances (light filter substances) that are liquid or crystals at a room temperature, and can absorb ultraviolet radiation and release absorbed energy in the form of long-wave radiation such as heat. Generally, content of the UV filter substances is 0.05-50 wt%, preferably 0.5-40 wt%.

The UV filter substances can be oil-soluble or water-soluble.

Appropriate oil-soluble substances comprise:

·3-benzylidenecamphor and derivatives thereof, such as 3-(4-methylbenzylidene)camphor;
·4-aminobenzoic acid derivatives, preferably 2-ethylhexyl p-dimethylaminobenzoate, 2-octyl 4-(dimethylamino) benzoate and pentyl 4-(dimethylamino)benzoate;
·esters of cinnamic acid, preferably 2-ethylhexyl 4-methoxycinnamate, propyl 4-methoxycinnamate, isopentyl 4-methoxycinnamate, 2-ethylhexyl 2-cyano-3,3-diphenylacrylate (octocrilene);
·esters of salicylic acid, preferably 2-ethylhexyl salicylate, homosalate and menthyl salicylate;
·benzophenone derivatives, preferably 2,2'-dihydroxy-4,4'-dimethoxybenzophenone, 2-hydroxy-4-methoxybenzophenone and 2,2'-dihydroxy-4-methoxybenzophenone;
·benzyl malonate, preferably bis(2-ethylhexyl) 2-(4-methoxybenzyl)malonate;
·triazine derivatives, for example, 2,4,6-tri-(p-2-ethylhexyl aniline)-1,3,5-triazine and dioctyl butamido triazone (Iscotrizinol);
·benzoylmethane derivatives, preferably 4-t-butyl-4'-methoxydibenzoylmethane;
·carbonyl polycyclic compounds, such as keto-tricyclo[5.2.1.0] decane derivatives.

Embodiments of appropriate water-soluble substances comprise:

·2-phenylbenzimidazole-5-sulfonic acid and alkali metal, alkaline-earth metal, ammonium, alkylammonium, alkanol and glucosamine salts thereof;
·disodium salt of 2,2'-bis-(1,4-phenylene)-1H-benzimidazole-4,6-disulfonic acid;
·sulfonic acid derivatives of benzophenone, preferably 2-hydroxy-4-methoxybenzophenone-5-sulfonic acid and salts thereof; and
·sulfonic acid derivatives of 3-benzylidene camphor, preferably benzylidene camphor sulfonic acid and 2-methyl-5-(2-oxo-3-bornylene)sulfonic acid and salts thereof.

Typical examples of the particularly appropriate UV-A light filter substance comprise derivatives of benzoylmethane, such as 4-tert-butyl-4'-methoxy-dibenzoylmethane, hexyl 2-(4-diethylamino-2-hydroxybenzoyl)-benzoate, 1-phenyl-3-(4'-isopropylphenyl)propane-1,3-dione and enamine compounds. Certainly, the UV-A and UV-B filter substances can be mixed, for example, a combination of the diphenylmethane derivative and/or benzoylmethane derivative 4-t-butyl-4'-methoxydibenzoylmethane and the cinnamic acid derivative, wherein the cinnamic acid derivative is preferably 2-ethylhexyl 2-cyano-3,3-diphenylacrylate (octocrilene) and/or 2-ethylhexyl trans-4-methoxycinnamate and/or isopentyl 4-methoxycinnamate and/or propyl 4-methoxycinnamate. The cinnamate derivatives of the combinations can be water-soluble filter substances such as 2-phenylbenzimidazole-5-sulfonic acid and alkali metal, alkaline-earth metal, ammonium, alkylammonium, alkanol and glucosamine ammonium salts thereof and/or 4-(2-oxo-3-bornylenemethyl) benzenesulfonic acid and 2-methyl-5-(2-oxo-3-bornylene) sulfonic acid and alkali metal, alkaline-earth metal, ammonium, alkylammonium, alkanol and glucosamine ammonium salts thereof.

In one preferred implementation solution, the (cosmetic or pharmaceutical) preparation in the present invention comprises at least one extra UV-absorbing compound, selected from groups comprising:

·camphor benzalkonium methosulfate
·terephthalylidene dicamphor sulfonic acid and salts

·homomenthyl salicylate
·benzylidene camphor sulfonic acid and salts
·2-ethylhexyl 2-cyano-3,3-diphenylacrylate
·ethyl p-aminobenzoate
·isoamyl 4-Methoxycinnamate
·2-phenylbenzimidazole sulfonic acid and salts
·2,4,6-tri-(p-2-ethylhexyl aniline)-1,3,5-triazine
·2-(2H-benzotriazol-2-yl)-4-methyl-6-{2-methyl-3-{1,3,3,3-tetramethyl-1-[(trimethylsilyl)oxy]-disiloxanyl}-propyl} phenol
·methylene bis-benzotriazolyl tetramethylbutylphenol
·4,4'-[(6-[4-(1,1-dimethyl)-aminocarbonyl]phenylamino)-1,3,5-triazine-2,4-diyl]diimino]-bis-(2-ethylhexyl benzoate)
·3-(4'-methylbenzylidene)-D,L-camphor
·3-benzylidene camphor
·2-ethylhexyl salicylate
·2-ethylhexyl 4-(dimethylamino)benzoate
·4-hydroxy-4-methoxybenzophenone-5-sulfonic acid and salts thereof
·benzylidenemalonate-polysiloxane
·menthyl anthranilate
·polyacrylamidomethyl benzylidene camphor
-2-ethylhexyl 4-methoxycinnamate
or mixtures thereof.

When applied to human skin, the composition in the present invention can be used with a skin brightener to enhance a retarding effect of hyperpigmentation. Appropriate skin brighteners used in the present invention comprise: kojic acid derivatives (preferably kojic dipalmitate), arbutin, ascorbic acid and ascorbic acid derivatives (preferably magnesium ascorbyl phosphate), hydroquinone and hydroquinone derivatives, sclareolide, amino acids (preferably cyclohexyl carbamate, N-acetyl tyrosine and derivatives thereof and undecylenoyl phenylalanine), sulfur-containing molecules (preferably glutathione, cysteine, thiourea derivatives and lipoic acid), $\alpha$-hydroxy acids (preferably citric acid, lactic acid, malic acid and salts and esters thereof), gluconic acids, chromone derivatives (preferably aloesin), 1-aminoethyl phosphinic acid, flavonoids compounds, ellagic acid, nicotinamide, thujaplicin and derivatives thereof, triterpenes (preferably maslinic acid), sterols (preferably ergosterol), benzofuranones (preferably senkyunolide), 4-vinyl guaiacol, 4-ethyl guaiacol, zinc salts (preferably zinc chloride or zinc gluconate), diacids (preferably octadecene diacid and/or azelaic acid), nitric oxide synthesis inhibitors (preferably L-nitroarginine and derivatives thereof, 2,7-dinitroindazole or L-thiocitrulline), metal chelating agents (preferably $\alpha$-hydroxy fatty acids, phytic acid, cholic acid, bile extract, humic acid, EGTA, EDTA and derivatives thereof), soymilk and extract thereof, retinoic acid compounds, serpin inhibitors, other synthesized or extracted natural active ingredients (preferably used in the form of plant extracts, such as bearberry extract, grape extract, mulberry extract, pachyrhizus extract, rice extract, papaya extract, curcuma extract, nut grass extract, licorice root extract (preferably glabridin or licochalcone A), artocarpus extract, rumex extract, pinus (pine) extract, vitis extract or stilbenoid derivatives separated or concentrated from the vitis extract, *Saxifraga sarmentosa* extract, *Scutellaria baicalensis* extract, microalgae extract (preferably *Tetraselmis tetrathele* extract) and ginseng extract.

The present invention will be described in detail below through embodiments. However, it can be understood that related embodiments are not used for limiting the protection scope.

In the embodiments below:
Amounts of reactants and products are measured by liquid chromatography (Agilent HPLC 1260).

The conversion and selectivity of the reaction are calculated by formulas as follows:

Conversion=(mole number of added raw materials- mole number of residual raw materials in the product)/the mole number of added raw materials$\times$100%.

Selectivity=actual mole number of a target product/theoretical mole number of the target product$\times$100%.

Unless otherwise specified, used raw materials are all commercially available products, and the room temperature is 25$\pm$5°C.

Embodiment 1

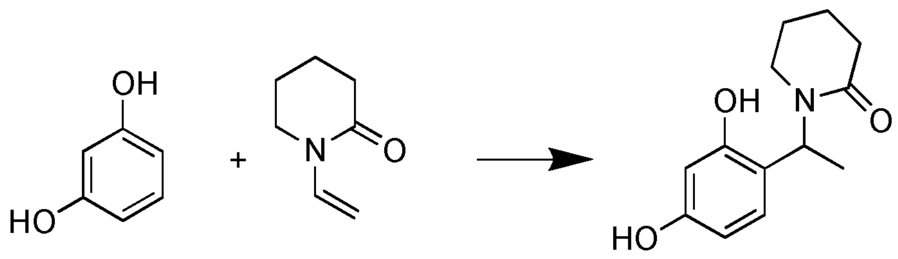

250 g of toluene, 1.0 g of a catalyst p-toluenesulfonic acid, 110 g of resorcinol and 125 g of 1-ethenylpiperidin-2-one were added into a 1000 ml four-necked flask; the flask was heated; the components reacted at 90°C for 10 hours; the liquid chromatography detection result showed the completion of the reaction; and after the solvent was removed at a reduced pressure, an ethyl acetate/petroleum ether mixed solvent was added for recrystallization, thereby obtaining the product with the yield of 90% and the purity of 99%.

Embodiment 2

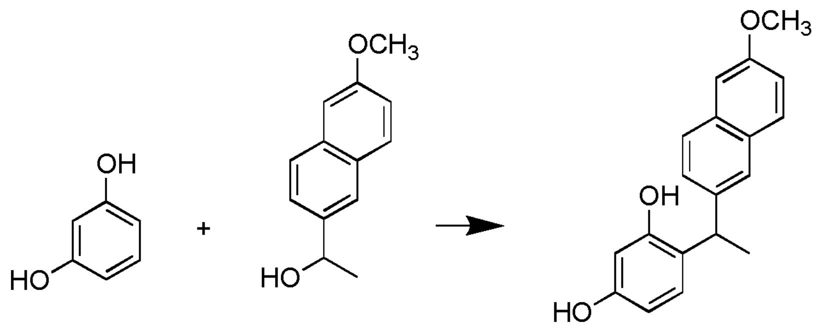

250 g of toluene, 1.0 g of a catalyst p-toluenesulfonic acid, 110 g of resorcinol and 202 g of 1-(6-methoxynaphthalen-2-yl)ethan-1-ol were added into a 1000 ml four-necked flask; the flask was heated; the components reacted at 90°C for 10 hours; the liquid chromatography detection result showed the completion of the reaction; and after the solvent was removed at a reduced pressure, an ethyl acetate/petroleum ether mixed solvent was added for recrystallization, thereby obtaining the product with the yield of 92% and the purity of 99%.

Embodiment 3

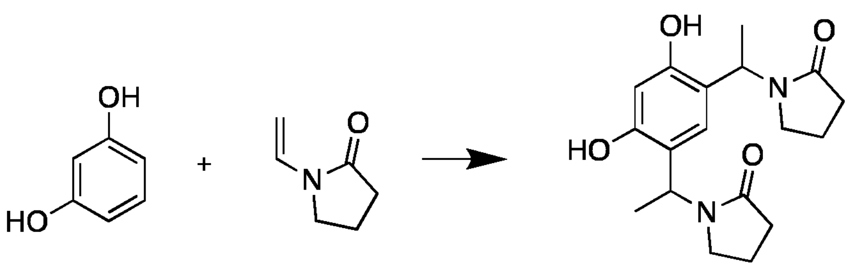

250 g of toluene, 1.0 g of a catalyst p-toluenesulfonic acid, 110 g of resorcinol and 222 g of N-vinyl-2-pyrrolidinone were added into a 1000 ml four-necked flask; the flask was heated; the components reacted at 90°C for 10 hours; the liquid chromatography detection result showed the completion of the reaction; and after the solvent was removed at a reduced pressure, an ethyl acetate/petroleum ether mixed solvent was added for recrystallization, thereby obtaining the product with the yield of 85% and the purity of 96%.

Embodiment 4

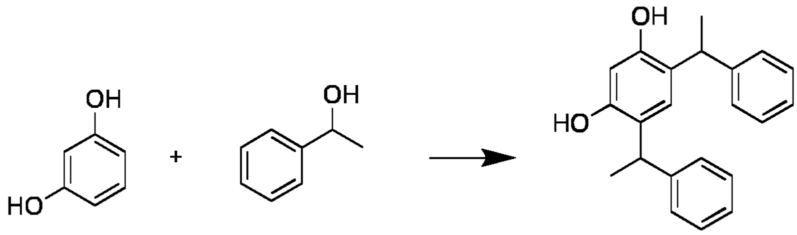

250 g of toluene, 1.0 g of a catalyst p-toluenesulfonic acid, 110 g of resorcinol and 244 g of 1-phenylethan-1-ol were added into a 1000 ml four-necked flask; the flask was heated; the components reacted at 90°C for 10 hours; the liquid chromatography detection result showed the completion of the reaction; after the solvent was removed at a reduced pressure, the product was separated and purified through a silica gel column (leaching with an ethyl acetate/petroleum ether mixed solvent); and the solvent was concentrated and removed, thereby obtaining the product with the yield of 82% and the purity of 97%.

Embodiment 5

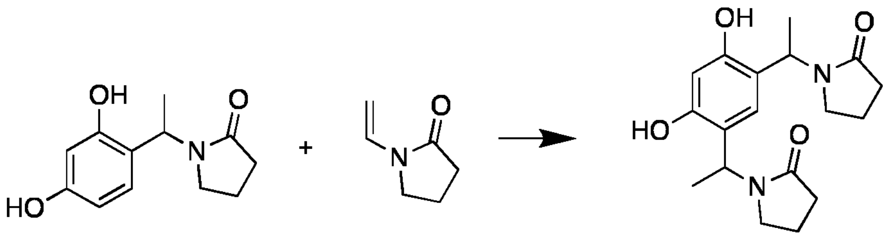

150 g of toluene, 0.50 g of a catalyst p-toluenesulfonic acid, 110 g of a resorcinol pyrrolidinone derivative and 56 g of N-vinyl-2-pyrrolidinone were added into a 1000 ml four-necked flask; the flask was heated; the components reacted at 90°C for 10 hours; the liquid chromatography detection result showed the completion of the reaction; and after the solvent was removed at a reduced pressure, an ethyl acetate/petroleum ether mixed solvent was added for recrystallization, thereby obtaining the product with the yield of 84% and the purity of 97%.

Embodiment 6

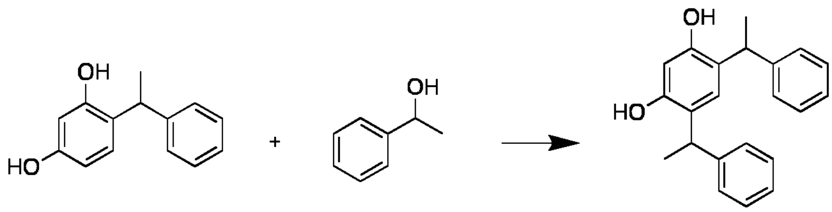

250 g of toluene, 1.0 g of a catalyst p-toluenesulfonic acid, 214 g of a resorcinol benzene substituted ethyl derivative and 122 g of 1-phenylethan-1-ol were added into a 1000 ml four-necked flask; the flask was heated; the components reacted at 90°C for 10 hours; the liquid chromatography detection result showed the completion of the reaction; after the solvent was removed at a reduced pressure, the product was separated and purified through a silica gel column (leaching with an ethyl acetate/petroleum ether mixed solvent); and the solvent was concentrated and removed, thereby obtaining the product with the yield of 80% and the purity of 95%.

Compounds in Tables 1-3 below are prepared with reference to the methods in the above embodiments.

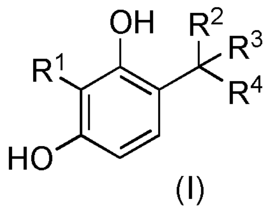

(I)

Table 1: compounds of the structural formula I

| No. | $R^1$ | $R^2$ | $R^3$ | $R^4$ | m/z |
|---|---|---|---|---|---|
| I-a | H | 2-oxopiperidin-1-yl | H | $CH_3$ | 235.1 |
| I-b | H | 6-methoxy-2-naphthalenyl | H | $CH_3$ | 294.1 |
| I-c | H | 2-oxopiperidin-1-yl | H | $CH_2CH_3$ | 249.1 |
| I-d | H | 2-oxopyrrolidin-1-yl | H | $CH_2CH_3$ | 235.1 |
| I-e | H | 2-oxopiperidin-1-yl | H | $CH_2CH_2CH_3$ | 263.2 |
| I-f | H | 2-oxopyrrolidin-1-yl | H | $CH_2CH_2CH_3$ | 249.1 |
| I-g | H | 2-oxoazepan-1-yl | H | $CH_3$ | 249.1 |
| I-h | H | 2-oxopyrrolidin-1-yl | H | $CH_3$ | 221.1 |
| I-i | H | 2-oxoazepan-1-yl | H | $CH_2CH_3$ | 263.2 |
| I-j | H | 2-oxoazepan-1-yl | H | $CH_2CH_2CH_3$ | 277.2 |
| I-k | H | 2-oxopyrrolidin-1-yl | H | H | 207.1 |
| I-l | H | 2-oxopiperidin-1-yl | H | H | 221.1 |
| I-m | H | 2-oxoazepan-1-yl | H | H | 235.1 |
| I-n | H | acetamido | H | $CH_3$ | 195.1 |
| I-o | H | 2-carboxy-5-oxopyrrolidin-1-yl | H | $CH_3$ | 265.1 |
| I-p | H | 2-(methoxycarbonyl)-5-oxopyrrolidin-1-yl | H | $CH_3$ | 279.1 |
| I-q | H | 2-(methylcarbamoyl)-5-oxopyrrolidin-1-yl | H | $CH_3$ | 278.1 |
| I-r | H | formamido | H | $CH_3$ | 181.1 |
| I-s | $CH_3$ | 2-oxopiperidin-1-yl | H | $CH_3$ | 249.1 |
| I-t | $CH_3$ | 2-oxoazepan-1-yl | H | $CH_3$ | 263.2 |
| I-u | $CH_3$ | 2-oxopyrrolidin-1-yl | H | $CH_3$ | 235.1 |
| I-v | H | phenyl | H | 1-n-dodecyl-2-oxoazepan-3-yl | 479.3 |
| I-w | H | phthalimido | H | $CH_3$ | 283.1 |

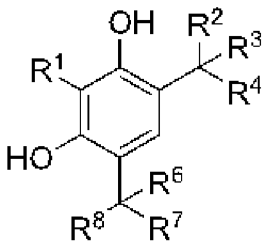

(VI)

Table 2: compounds of the structural formula VI

| No. | $R^1$ | $R^2$ | $R^3$ | $R^4$ | $R^6$ | $R^7$ | $R^8$ | m/z |
|---|---|---|---|---|---|---|---|---|
| V-a | H | phenyl | H | $CH_3$ | phenyl | H | $CH_3$ | 318.2 |
| V-b | H | 2-oxoazepan-1-yl | H | $CH_3$ | 2-oxoazepan-1-yl | H | $CH_3$ | 388.2 |
| V-c | H | 2-oxopyrrolidin-1-yl | H | $CH_3$ | 2-oxopyrrolidin-1-yl | H | $CH_3$ | 32.2 |
| V-d | H | 2-oxopiperidin-1-yl | H | $CH_3$ | 2-oxopiperidin-1-yl | H | $CH_3$ | 360.2 |
| V-e | $CH_3$ | 2-oxopiperidin-1-yl | H | $CH_3$ | 2-oxopiperidin-1-yl | H | $CH_3$ | 374.2 |
| V-f | H | acetamido | H | $CH_3$ | acetamido | H | $CH_3$ | 280.1 |
| V-g | H | formamido | H | $CH_3$ | formamido | H | $CH_3$ | 252.1 |
| V-h | H | 2-carboxy-5-oxopyrrolidin-1-yl | H | $CH_3$ | 2-carboxy-5-oxopyrrolidin-1-yl | H | $CH_3$ | 420.2 |
| VI-a | H | 2-oxopyrrolidin-1-yl | H | $CH_3$ | phenyl | H | $CH_3$ | 325.2 |

Table 3: Nuclear magnetic resonance data of compounds I-a to I-g

| Compound No. | Compound structure | H-NMR |
|---|---|---|
| I-a | 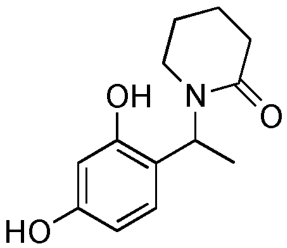 | $^1$H NMR (500MHz, DMSO-d6) δ 1.33 (d, J=7.1Hz, 3H), 1.49-1.62 (m, 4H), 2.16-2.27 (m, 2H), 2.70-2.75(m,1H),3.04-3.09 (m,1H), 5.71(q, J=7.1Hz, 1H), 6.18-6.21 (m,2H), 7.01 (d, J=8.1Hz, 1H), 9.29 (s, 1H), 9.43 (s, 1H). |
| I-b | 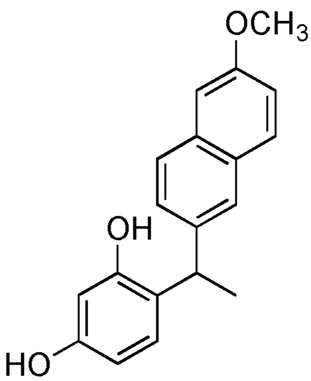 | $^1$H NMR (500MHz, DMSO-d6) δ 1.53 (d, J=7.3Hz, 3H), 3.84 (s, 3H), 4.45 (q, J=7.3Hz, 1H), 6.16 (dd, J=8.5, 2.5Hz, 1H), 6.27 (d, J=2.5Hz, 1H), 6.85 (d, J=8.5Hz, 1H), 7.10 (dd, J=9.0, 2.5Hz, 1H), 7.23 (d, J=2.0Hz, 1H), 7.30 (dd, J=8.5, 2.0Hz, 1H), 7.61 (s, 1H), 7.67 (d, J=8.5Hz,1H), 7.74 (d, J=9.0Hz,1H), 9.01 (s, 1H), 9.18 (s, 1H). |
| I-c | 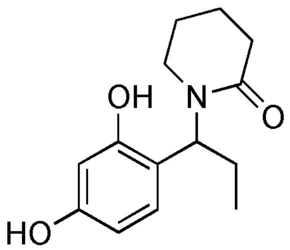 | $^1$H NMR (500MHz, DMSO-d6) δ 0.81 (t, J=7.0Hz, 3H), 1.46-1.72 (m, 4H), 1.76-1.84 (m, 1H), 1.86-1.95 (m, 1H), 2.20-2.34 (m, 2H), 2.82-2.86(m, 1H), 3.08-3.13 (m, 1H), 5.47 (t, J=7.5Hz, 1H), 6.19-6.21 (m, 2H), 7.01 (d, J=8.0Hz, 1H), 9.23 (s, 1H), 9.36 (s, 1H). |
| I-d | 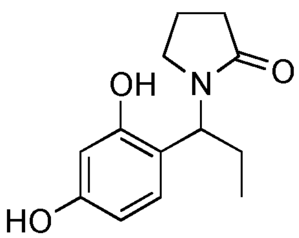 | $^1$H NMR (500MHz, DMSO-d6) δ 0.79 (t, J=7.5Hz, 3H), 1.76-1.91 (m, 4H), 2.14-2.28 (m, 2H), 2.95-2.99 (m, 1H), 3.26-3.30 (m, 1H), 5.04 (t, J=7.5Hz, 1H), 6.19(dd, J=8.5, 2.5Hz, 1H), 6.25 (d, J=2.5Hz, 1H), 6.97(d, J=8.5Hz, 1H), 9.19 (s, 1H), 9.21 (s, 1H). |

(continued)

| Compound No. | Compound structure | H-NMR |
|---|---|---|
| I-e | 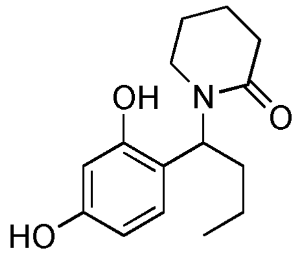 | 1H NMR (500MHz, DMSO-d6) δ 0.89 (t, J=7.0Hz, 3H), 1.15-1.25 (m, 2H), 1.45-1.52 (m, 1H), 1.58-1.77 (m, 4H), 1.85-1.93 (m, 1H), 2.19-2.34(m, 2H), 2.83-2.87 (m, 1H), 3.08-3.14 (m, 1H), 5.59 (t, J=7.5Hz, 1H), 6.19-6.21 (m, 2H), 7.02 (d, J=9.0Hz, 1H), 9.23 (s, 1H), 9.36 (s, 1H). |
| I-f | 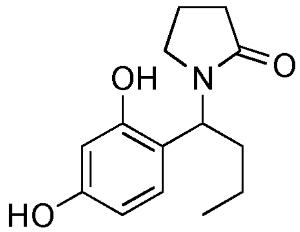 | 1H NMR (500MHz, DMSO-d6) δ 0.88 (t, J=7.5Hz, 3H), 1.18-1.24 (m, 2H), 1.69-1.91 (m, 4H), 2.13-2.26 (m, 2H), 2.95-3.00 (m, 1H), 3.26-3.30 (m, 1H), 5.16 (t, J=7.5Hz,1H), 6.19(dd, J=8.0, 2.5Hz, 1H), 6.25 (d, J=2.5Hz, 1H), 6.97(d, J=8.0Hz, 1H), 9.19 (s, 1H), 9.20 (s, 1H). |
| I-g | 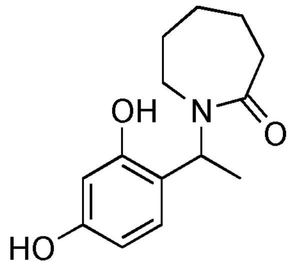 | 1H NMR (500MHz, DMSO-d6) δ 1.11-1.21 (m, 1H), 1.21-1.28 (m, 1H), 1.30 (d, J=7.0Hz, 3H), 1.46-1.61 (m, 4H), 2.37-2.46 (m, 2H), 3.02-3.18 (m, 2H), 5.64 (q, J=7.0Hz, 1H), 6.19-6.22 (m, 2H), 7.02 (d, J=8.0Hz, 1H), 9.19 (s, 1H), 9.21 (s, 1H). |
| V-a | 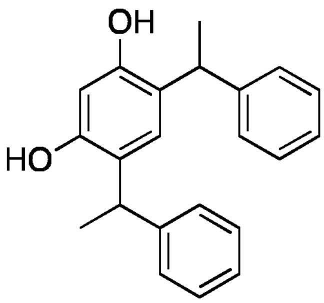 | 1H NMR (500MHz, DMSO-d6) δ1.40-1.44 (m, 6H), 4.31 (q, J=7.0Hz, 2H), 6.29 (s, 1H), 6.90 (d, J=6.0Hz, 1H), 7.08-7.22 (m, 10H), 8.96 (s, 2H). |
| V-c | 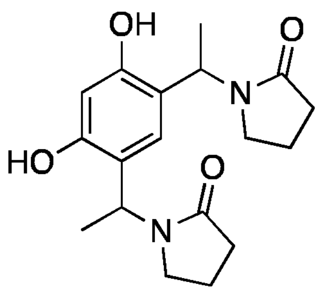 | 1H NMR (500MHz, DMSO-d6) δ1.36 (d, J=7.5Hz, 6H), 1.78-1.89 (m, 4H), 2.15-2.25 (m, 4H), 2.85-2.94 (m, 2H), 3.26-3.35 (m, 2H), 5.25 (m, 2H), 6.31 (s, 1H), 6.98 (s, 1H), 9.30 (s, 1H), 9.30(s, 1H). |

Application test case: evaluation of inhibitory activity of tyrosinase

1.1 Test method A: Testing inhibitory activity of samples on tyrosinase by a light absorption method

The compound in the present invention was respectively prepared into solutions having 5 concentrations, e.g., 5 μg/mL, 3 μg/mL, 1 μg/mL, 0.5 μg/mL and 0.01 μg/mL; positive control kojic acid was respectively prepared into solutions

having concentrations of 25 μg/mL, 20 μg/mL, 10 μg/mL, 7.5 μg/mL, 5 μg/mL and 2.5 μg/mL; 50 μL of the two kinds of solutions having 5 concentrations were taken respectively; 1 mL solution was prepared from 950 μL of a phosphate buffer solution having a pH value of 6.8; 1 mL of 0.1 mg/mL tyrosine was added; then the 1 mL tyrosinase (200 U/mL) prepared from the phosphate buffer solution having the pH value of 6.8 was added; incubation was conducted at 37°C for 20 min; and an absorbance value was determined at 490 nm.

$$\text{Enzyme activity inhibition ratio} = [(A2-A1)-(B2-B1)]/(A2-A1)\times 100\%$$

A1 is an absorption value at 0 min when an inhibitor is not added; A2 is an absorption value within 20 min when the inhibitor is not added;

B1 is an absorption value at 0 min when the inhibitor is added; and B2 is an absorption value within 20 min when the inhibitor is added.

Test results show that, the compound in the present invention has an excellent inhibitory effect of inhibiting the tyrosinase at IC50 of 10 μM; the preferred compounds I-a, I-b, I-c, I-d, I-e, I-f, I-g, I-h, V-a, V-b and V-c have excellent inhibitory effects at 0.05-1 μM; and the control sample kojic acid has IC50 of 47.0 μM. The above results indicate that, the compounds in the present invention have excellent tyrosinase inhibitory activity. Moreover, the compounds in the present invention can serve as tyrosinase inhibitors alone or in combination to be used for inhibition of enzymatic browning of fruits and vegetables, whitening in whitening cosmetics, and preparation of various pharmaceutical preparations for preventing and treating human pigmentation diseases caused by excessive melanin, melanoma and other diseases with needs of inhibiting tyrosinase activity.

1.3 Test method B:

1.3.1 Cytotoxicity test

1) Cell inoculation: cells were inoculated to a 96-well plate at inoculum density of $1\times 10^4$ cells per well, and incubation was conducted in a 5% $CO_2$ incubator at 37°C overnight.

2) Experimental grouping: a zero setting group, a control group, a positive control group and a sample group were set in the experiment; and in the sample group, each sample was set with different concentration gradients, and 3 repeat holes were set at each concentration gradient.

3) Solution preparation: sample working solutions of different concentrations were prepared according to a test concentration setting table.

4) Drug administration: drug administration was conducted when a cell planking ratio was up to 40-60% in the 96-well plate; 200 μL of a culture solution containing 10% PBS (Gibco) was added into each well of the control group; 200 μL of a culture solution containing 10% DMSO was added into each well of the positive control group; 200 μL of a culture solution containing a sample of a corresponding concentration was added into each well of the sample group; cell inoculation was not conducted in the zero setting group, and only 200 μL of a cell culture solution was added; and after the drug administration was completed, the 96-well plate was cultured in the incubator (37°C, 5% $CO_2$).

5) Detection: within 24 h after cell inoculation and culture, the supernatant was removed; an MTT working solution (0.5 mg/mL) was added; inoculation was conducted in the dark at 37°C for 4 h; the supernatant was removed after inoculation was completed; 100 μL of DMSO was added into each well; and a value OD was read at 490 nm.

6) Calculation of relative cell viability:

$$\text{Relative cell viability} = \frac{\text{OD of the sample well} - \text{OD of the zero setting well}}{\text{OD of the solvent control well} - \text{OD of the zero setting well}} * 100\%$$

Examples of test data of cell viability:

| Sample concentration (%, g/ml) | 0.5 | 0.25 | 0.125 | 0.03125 | 0.015625 | 0.007813 |
|---|---|---|---|---|---|---|
| I-g cell viability | 107.9874 | 120.9409 | 113.8122 | 107.8966 | 120.6987 | 117.8165 |
| Cell viability* | 96.77 | 106.07 | 100.10 | 101.15 | 114.68 | 106.32 |
| *Control sample is phenylethyl resorcinol. | | | | | | |

The cell viability data show that, the compound structure in the present invention has low cytotoxicity.

1.3.2 Cell melanogenesis inhibition test

According to the cytotoxicity test data, cell melanogenesis inhibition test was conducted in a cytotoxicity permissive range. Samples were grouped according to different concentrations; a detection index was melanin content; a detection model was melanocyte; detection was conducted by a developing process; cells in a logarithmic growth phase were collected and inoculated to a 24-well plate at cell density of $2\times10^5$ cells per bottle; after the cells were cultured in an incubator (37°C, 5% $CO_2$) for 24 h, a drug was added according to concentration requirements in accordance with the cytotoxicity results; untreated cells served as blank control; 3 parallels were set for each group; the cells were continuously cultured in the incubator (37°C, 5% $CO_2$) for 48 h after the drug was added; the supernatant was removed; 1 mL of 1M NaOH containing 10% DMSO was added; the cells were incubated in a constant temperature oven at 60°C for 1 h; 200 μL of the cells were transferred from each well to the 96-well plate after the room temperature was restored; the 1M NaOH containing 10% DMSO served as blank control; an absorbance value was read at 405 nm; and a relative inhibition ratio of cell melanin was calculated.

$$\text{Cell melanogenesis inhibition ratio\%} = \left(1-\frac{\text{OD of the drug administration well}-\text{OD of the blank well}}{\text{OD of the control well}-\text{OD of the blank well}}\right)*100\%$$

Results of the cell melanogenesis inhibition ratio

| Sample | Concentration (%, g/ml) | Cell melanogenesis inhibition ratio (%) |
|---|---|---|
| I-a | 0.10 | 71.10 |
| I-a | 0.050 | 50.22 |
| I-a | 0.025 | 18.55 |
| I-d | 0.10 | 67.80 |
| I-d | 0.050 | 50.40 |
| I-d | 0.025 | 33.54 |
| I-e | 0.10 | 34.99 |
| I-f | 0.05 | 48.15 |
| I-g | 0.05 | 43.26 |
| I-h | 0.10 | 46.30 |
| V-a | 0.010 | 73.80 |
| V-a | 0.0050 | 47.60 |
| V-a | 0.0025 | 28.89 |
| V-b | 0.10 | 32.05 |
| V-c | 0.10 | 54.85 |
| PC* | 0.30 | 66.37 |
| *Notes: the PC sample is the kojic acid. | | |

Test results show that, the compounds I-a, I-d, I-e, I-f, I-g, I-h, V-a, V-b and V-c in the present invention have cell

melanogenesis inhibition effects at a lower concentration (not exceeding 0.10% g/ml); preferably, the I-a, I-d and V-a show the cell melanogenesis inhibition effects better than that of the kojic acid, even can inhibit cell melanogenesis at an extremely low concentration.

The above results show that, the compounds in the present invention have excellent tyrosinase inhibitory activity; and the compounds in the present invention can serve as the tyrosinase inhibitors alone or in combination to be used for whitening in whitening cosmetics and for preparation of various pharmaceutical preparations for preventing and treating human pigmentation diseases caused by excessive melanin, melanoma and other diseases with needs of inhibiting tyrosinase activity.

Although the present invention has been disclosed above through the preferred embodiments, the embodiments are not used for limiting the present invention. Various changes and modifications may be made by those skilled in the art without departing from the spirit and scope of the present invention. Therefore, the protection scope of the present invention should be defined based on the claims.

## Claims

**1.** A kind of compounds of formula A,

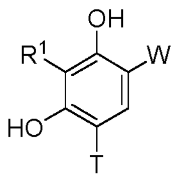

A

,

wherein W is

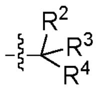

,

and T is H,

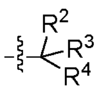

or

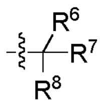

;

wherein $R^1$ is selected from H, optionally substituted alkyl and optionally substituted aryl alkyl;
$R^2$ and $R^6$ can be identical or different, and are independently selected from optionally substituted aryl, optionally substituted heteroaryl, optionally substituted alkyl, optionally substituted amide and optionally substituted ester respectively; $R^3$, $R^4$, $R^7$ and $R^8$ can be identical or different, and are independently selected from hydrogen, optionally substituted alkyl, optionally substituted cycloalkyl and optionally substituted amide respectively; $R^3$ and $R^4$ can be connected to carbon atoms jointly connected to form a ring; $R^7$ and $R^8$ can be connected to carbon atoms jointly connected to form a ring, wherein the amide group is preferably $-NHCOCH_3$, -NHCOH, $-NHCOCH_2CH_3$, $-NHCOCH_2CH_2CH_3$, $-NHCOCH(CH_3)_2$, $-NHCOCH(CH_2)_2$, $-N(COCH_3)_2$, $-CONH_2$, $-CON(CH_3)_2$, $-CONHCH_3$, $-CONHCH_2CH_3$, - $CON(CH_2CH_3)_2$, $-CONHCH(CH_3)_2$, $-CONHCH_2CH_2CH_3$, $-CONHCH_2CH_2CH_2CH_3$, phthaloyl, succinimido, glutarimido, maleimido,

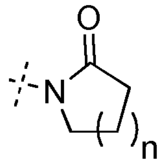 and 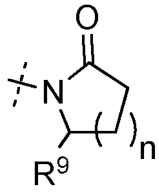 ,

wherein $R^9$ is selected from optionally substituted alkyl or optionally substituted aryl, carboxyl or a salt thereof, cyano, optionally substituted amide and optionally substituted ester; n is selected from a natural number from 1 to 6; and the ester is preferably one of $-COOCH_3$, $-COOCH_2CH_3$, $-COOCH(CH_3)_2$, $-COOCH_2CH_2CH_3$ and $-COOCH_2CH_2CH_2CH_3$;
or cosmetically or pharmaceutically acceptable salt, stereoisomers or a mixture of stereoisomers of any proportion, particularly enantiomers or a mixture of enantiomers, and more particularly a racemic mixture.

**2.** The compound according to claim 1, wherein the compound A is a compound of formula (I),

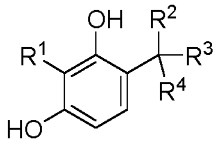

(I),

wherein $R^1$ to $R^4$ are defined as claim 1.

**3.** The compound according to claim 1 or 2, wherein the compound A is a compound of formula (Ia),

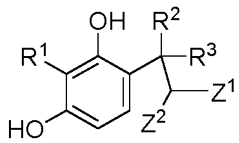

(Ia)

,

wherein $R^1$ to $R^3$ are defined as claim 1 or 2; $Z^1$ and $Z^2$ can be identical or different, and are independently selected from hydrogen, optionally substituted alkyl, optionally substituted cycloalkyl and optionally substituted amide respectively; $Z^1$ and $Z^2$ can be connected with carbon atoms jointly connected to form a ring; and the ring can be substituted.

**4.** The compound according to claim 1, wherein the compound A is a compound of formula (V),

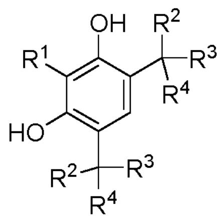

(V),

wherein $R^1$ to $R^4$ are defined as claim 1.

**5.** The compound according to claim 4, wherein the compound A is a compound of formula (Va),

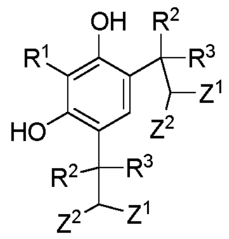

,

wherein $R^1$ to $R^3$ are defined as claim 4; $Z^1$ and $Z^2$ can be identical or different, and are independently selected from hydrogen, optionally substituted alkyl, optionally substituted cycloalkyl and optionally substituted amide respectively; $Z^1$ and $Z^2$ can be connected with carbon atoms jointly connected to form a ring; and the ring can be substituted.

6. The compound according to claim 1, wherein the compound A is a compound of formula (VI),

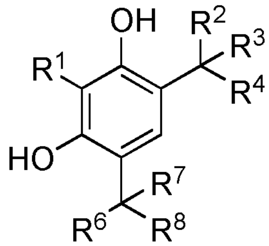

(VI),

wherein $R^1$ to $R^4$ and $R^6$ to $R^8$ are defined as claim 1.

7. The compound according to claim 6, wherein the compound A is (VIa),

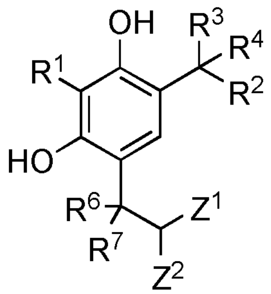

(VIa),

wherein $R^1$ to $R^4$ and $R^6$ to $R^7$ are defined as claim 1; $Z^1$ and $Z^2$ can be identical or different, and are independently selected from hydrogen, optionally substituted alkyl, optionally substituted cycloalkyl, and optionally substituted amide respectively; $Z^1$ and $Z^2$ can be connected with carbon atoms jointly connected to form a ring; and the ring can be substituted.

8. The compound according to any one of claims 1-7, selected from the following compounds:

N-(1-(2,4-dihydroxyphenyl) ethyl)-2-pyrrolidone;
N-(1-(2,4-dihydroxyphenyl) propyl)-2-pyrrolidone;
N-(1-(2,4-dihydroxyphenyl) ethyl)-2-piperidone;
N-(1-(2,4-dihydroxyphenyl) propyl)-2-piperidone;
N-(1-(2,4-dihydroxyphenyl) ethyl)-2- azepanone;
N-(1-(2,4-dihydroxyphenyl) propyl)-2- azepanone;
1,1'-((4,6-dihydroxy-1,3- phenylene) bis (ethane-1,1-diyl)) bis (pyrrolidin-2-one);
1,1'-((4,6-dihydroxy-1,3- phenylene) bis (propane-1,1-diyl)) bis (pyrrolidin-2-one);
1,1'-((4,6-dihydroxy-1,3- phenylene) bis (ethane-1,1-diyl)) bis (piperidin-2-one);

1,1'-((4,6-dihydroxy-1,3- phenylene) bis (propane-1,1-diyl)) bis (piperidin-2-one);
1,1'-((4,6-dihydroxy-1,3- phenylene) bis (ethane-1,1-diyl)) bis (azepan-2-one);
1,1'-((4,6-dihydroxy-1,3- phenylene) bis (propane-1,1-diyl)) bis (azepan-2-one);
4,6-bis (1-phenylethyl)-1,3-resorcinol.

**9.** A preparation method for preparing the compound of formula (I), wherein the compound of formula (II) reacts with the compound of formula (IVa) and a synthetic route is as follows:

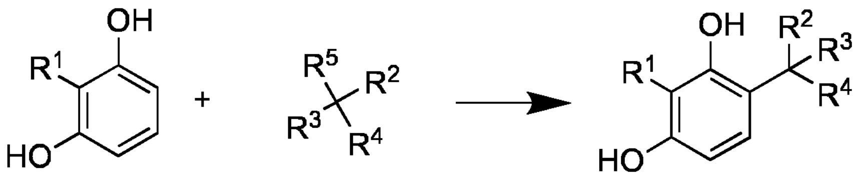

(II) (IVa) (I) ,

wherein $R^1$, $R^2$, $R^3$ and $R^4$ are defined as claim 1, wherein $R^5$ is selected from a leaving group, and the leaving group is preferably one of OH, $H_2O$, OTs, OMs, Cl, Br and I.

**10.** A preparation method for preparing the compound of formula (Ia), wherein the compound of formula (II) reacts with the compound of formula (IIIa) and a synthetic route is as follows:

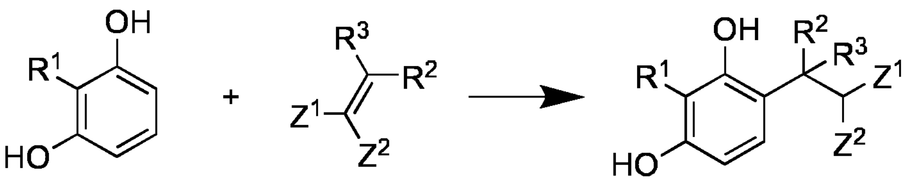

(II) (IIIa) (Ia) ,

wherein $R^1$, $R^2$ and $R^3$ are defined as claim 1, and $Z^1$ and $Z^2$ are defined as claim 3.

**11.** A preparation method for preparing the compound of formula (V), wherein the compound of formula (II) reacts with the compound of formula (IVa) and a synthetic route is as follows:

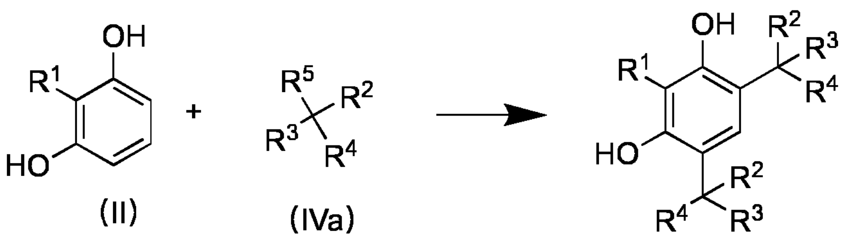

(II) (IVa) (V) ,

wherein $R^1$, $R^2$, $R^3$ and $R^4$ are defined as claim 1, wherein $R^5$ is selected from a leaving group, and the leaving group is preferably one of OH, $H_2O$, OTs, OMs, Cl, Br and I.

**12.** A preparation method for preparing the compound of formula (Va), wherein the compound of formula (II) reacts with the compound of formula (IIIa) and a synthetic route is as follows:

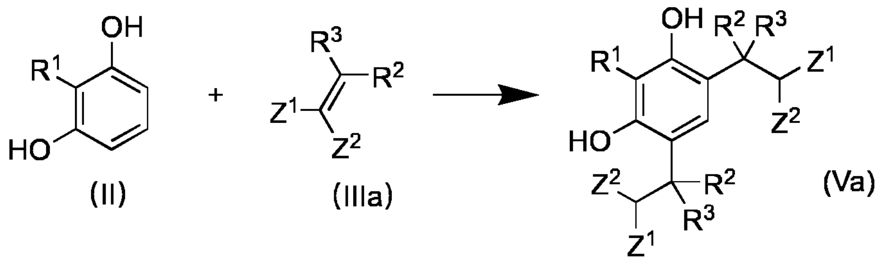
(II) (IIIa) (Va),

wherein $R^1$, $R^2$, $R^3$, $Z^1$ and $Z^2$ are defined as claim 5.

**13.** A preparation method for preparing the compound of formula (VI), wherein the compound of formula (I) reacts with the compound of formula (IVb) and a synthetic route is as follows:

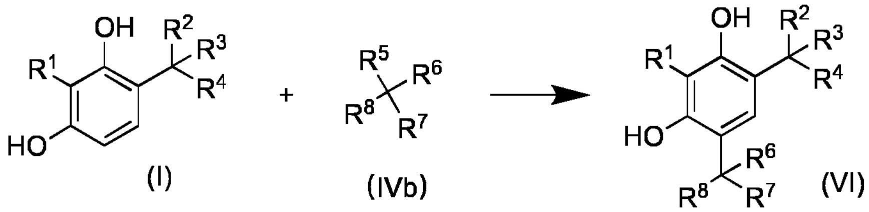
(I) (IVb) (VI),

wherein $R^1$, $R^2$, $R^3$, $R^4$, $R^6$, $R^7$ and $R^8$ are defined as claim 1, wherein $R^5$ is selected from a leaving group, and the leaving group is selected from one of OH, $H_2O$, OTs, OMs, Cl, Br and I.

**14.** A preparation method for preparing the compound of formula (VIa), wherein the compound of formula (I) reacts with the compound of formula (IIIb) and a synthetic route is as follows:

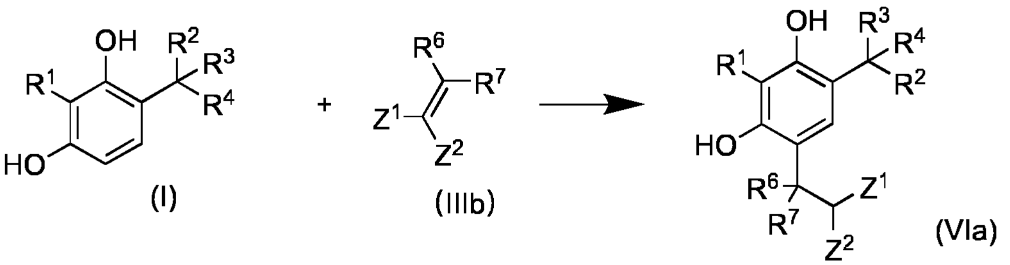
(I) (IIIb) (VIa),

wherein $R^1$, $R^2$, $R^3$, $R^4$, $R^6$ and $R^7$ are defined as claim 1, and $Z^1$ and $Z^2$ are defined as claim 7.

**15.** A pharmaceutical composition, comprising the compound according to any one of claims 1-8 or a pharmaceutically acceptable salt thereof.

**16.** A use of the compound according to any one of claims 1-8 in preparation of a drug for treating, preventing and/or alleviating hyperpigmentation.

**17.** A non-therapeutic and cosmetic use of the compound according to any one of claims 1-8 or a salt thereof in treating, preventing and/or alleviating hyperpigmentation.

**18.** A cosmetic composition, comprising the compound according to any one of claims 1-8 or a salt thereof.

**INTERNATIONAL SEARCH REPORT**

International application No.
**PCT/CN2023/085442**

**A. CLASSIFICATION OF SUBJECT MATTER**

C07D207/27(2006.01)i;A61Q 17/04(2006.01)i;A61Q 19/02(2006.01)i;A61K 8/00(2006.01)i;C07C37/00(2006.01)i

According to International Patent Classification (IPC) or to both national classification and IPC

**B. FIELDS SEARCHED**

Minimum documentation searched (classification system followed by classification symbols)

C07C、C07D、A61Q、A61K

Documentation searched other than minimum documentation to the extent that such documents are included in the fields searched

Electronic data base consulted during the international search (name of data base and, where practicable, search terms used)

CNTXT, DWPI, WPABS, VEN, ENTXT, Web of Science, STN, CNKI: 酪氨酸酶, 抑制剂, 1, 3-二酚, 黑色素, 黑素, 吡咯烷酮, 哌啶酮, 环己亚胺酮, tyrosinase, inhibitor, resorcinol, melanin, pyrrolidone, piperidone, cycloheximide.

**C. DOCUMENTS CONSIDERED TO BE RELEVANT**

| Category* | Citation of document, with indication, where appropriate, of the relevant passages | Relevant to claim No. |
|---|---|---|
| X | DATABASE REGISTRY [Online]. "CHEMICAL ABSTRACTS SERVICE" *COLUMBUS, OHIO, US,* 16 November 1984 (1984-11-16), retrieved from STN Database accession no. 65422-05-1 | 1, 2 |
| X | DATABASE REGISTRY [Online]. "CHEMICAL ABSTRACTS SERVICE" *COLUMBUS, OHIO, US,* 16 November 1984 (1984-11-16), retrieved from STN Database accession no. 80867-99-8 | 1-2 |
| X | WEN, Jingyun et al. "Hydroarylation of Styrenes with Electron-Rich Arenes Over Acidic Ion-Exchange Resins." *Synthetic Communications,* Vol. 44, No. 13, 29 May 2014 (2014-05-29), pages 1893-1903 ISSN: 0039-7911, see page 1895, Supplemental, pages 2-3 | 1-8, 10, 12, 14 |
| X | PRASHANT S. et al. "Microwave-Accelerated Alkylation of Arenes/Heteroarenes with Benzylic Alcohols Using Antimony(III) Chloride as Catalyst: Synthesis of O-Heterocycles" *Synlett,* Vol. 11, 15 June 2011 (2011-06-15), pages 1585-1591 ISSN: 0936-5214, see page 1586 | 1-9, 11, 13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 May 2023** | **31 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/085442**

**C. DOCUMENTS CONSIDERED TO BE RELEVANT**

| Category* | Citation of document, with indication, where appropriate, of the relevant passages | Relevant to claim No. |
|---|---|---|
| X | CN 113307725 A (LIAONING JINGFAN NEW MATERIAL CO., LTD.) 27 August 2021 (2021-08-27)<br>see description, paragraph 6 | 1, 4-7, 10, 12, 14 |
| X | CN 104523442 A (SUZHOU NANOHEALTH BIOTECH CO., LTD.) 22 April 2015 (2015-04-22)<br>see description, paragraphs 2 and 20 | 1-2, 17-18 |
| X | WO 0119323 A1 (KANSAI KOSO CO., LTD. et al.) 22 March 2001 (2001-03-22)<br>see description, pages 6-8 | 1-3, 15-18 |
| PX | ROULIER B. et al. "Resorcinol-based Hemiindigoid Derivatives as Human Tyrosinase Inhibitors and Melanogenesis Suppressors in Human Melanoma Cells"<br>*ChemRxiv,* Vol. 2022, 03 November 2022 (2022-11-03),<br>ISSN: 2573-2293,<br>see pages 10-11 | 1, 2, 15, 16 |
| A | CN 101175711 A (UNILEVER PLC) 07 May 2008 (2008-05-07)<br>see claim 1 | 1-18 |

Form PCT/ISA/210 (second sheet) (July 2022)

**INTERNATIONAL SEARCH REPORT**
**Information on patent family members**

International application No.
**PCT/CN2023/085442**

| Patent document cited in search report | | | Publication date (day/month/year) | Patent family member(s) | | | Publication date (day/month/year) |
|---|---|---|---|---|---|---|---|
| CN | 113307725 | A | 27 August 2021 | None | | | |
| CN | 104523442 | A | 22 April 2015 | None | | | |
| WO | 0119323 | A1 | 22 March 2001 | JPH | 11255637 | A | 21 September 1999 |
| CN | 101175711 | A | 07 May 2008 | JP | 2008533068 | A | 21 August 2008 |
| | | | | JP | 5225066 | B2 | 03 July 2013 |
| | | | | KR | 20070118122 | A | 13 December 2007 |
| | | | | CA | 2600816 | A1 | 21 September 2006 |
| | | | | MX | 2007011506 | A | 23 November 2007 |
| | | | | WO | 2006097223 | A1 | 21 September 2006 |
| | | | | ZA | 200707962 | B | 26 November 2008 |
| | | | | AU | 2006224815 | A1 | 21 September 2006 |
| | | | | TW | 200716534 | A | 01 May 2007 |
| | | | | TWI | 457142 | B | 21 October 2014 |
| | | | | US | 2006210497 | A1 | 21 September 2006 |
| | | | | EP | 1858835 | A1 | 28 November 2007 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012129260 A
- WO 2018001485 A
- WO 2015090850 A